(19) Europäisches Patentamt / European Patent Office / Office européen des brevets

(11) EP 2 269 589 B1

(12) **EUROPEAN PATENT SPECIFICATION**

(45) Date of publication and mention of the grant of the patent:
**25.12.2013 Bulletin 2013/52**

(21) Application number: **10186212.6**

(22) Date of filing: **30.03.2005**

(51) Int Cl.:
*A61K 9/16* (2006.01) *A61K 9/50* (2006.01)
*A61K 9/32* (2006.01) *A61K 47/32* (2006.01)
*A61P 3/12* (2006.01) *A61K 31/74* (2006.01)
*A61K 33/00* (2006.01) *A61K 45/06* (2006.01)

(54) **Ion binding polymers and uses thereof**

Ionen bindende Polymere und ihre Verwendung

Polymères se liant à des ions et leurs utilisations

(84) Designated Contracting States:
**AT BE BG CH CY CZ DE DK EE ES FI FR GB GR HU IE IS IT LI LT LU MC NL PL PT RO SE SI SK TR**

(30) Priority: **30.03.2004 US 814749**
**30.03.2004 US 813872**
**30.03.2004 US 814527**
**13.10.2004 US 965274**

(43) Date of publication of application:
**05.01.2011 Bulletin 2011/01**

(62) Document number(s) of the earlier application(s) in accordance with Art. 76 EPC:
**10152337.1 / 2 184 059**
**05732849.4 / 1 732 523**

(73) Proprietor: **Relypsa, Inc.**
**Redwood City CA 94063 (US)**

(72) Inventors:
- **Charmot, Dominique**
  **Campbell, CA 95008 (US)**
- **Chang, Han Ting**
  **Livermore, CA 94550 (US)**
- **Klaerner, Gerrit**
  **San Jose, CA 95124 (US)**
- **Cope, Michael James**
  **Berkeley,, 94702 (US)**
- **Liu, Mingjun**
  **Campbell, CA 95008 (US)**
- **Liu, Futian**
  **Sunnyvale, CA 94087 (US)**
- **Mong, Tony Kwok-Kong**
  **Sunnyvale, CA 94085 (US)**

(74) Representative: **Eisenführ, Speiser & Partner**
**Postfach 10 60 78**
**28060 Bremen (DE)**

(56) References cited:
- **KIM HO-JUNG ET AL: "THERAPEUTIC APPROACH TO HYPERKALEMIA", NEPHRON, S. KARGER AG, SWITZERLAND, vol. 92, no. SUPPL. 1, 1 October 2002 (2002-10-01), pages 33-40, XP009077430, ISSN: 0028-2766, DOI: 10.1159/000065375**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 269 589 B1

## Description

### BACKGROUND OF THE INVENTION

Potassium ($K^+$) is the most abundant intracellular cation, comprising ~ 35-40mEq/kg in humans. See Agarwal, R, et al. (1994) Gastroenterology 107: 548-571; Mandal, AK (1997) Med Clin North Am 81: 611-639. Only 1.5-2.5% of this is extracellular. Potassium is obtained through the diet, mainly through vegetables, fruits, meats and dairy products, with certain food such as potatoes, beans, bananas, beef and turkey being especially rich in this element. See Hunt, CD and Meacham, SL (2001) J Am Diet Assoc 101: 1058-1060; Hazell, T (1985) World Rev Nutr Diet 46: 1-123. In the US, intake is ~80mEq/day. About 80% of this intake is absorbed from the gastrointestinal tract and excreted in the urine, with the balance excreted in sweat and feces. Thus, potassium homeostasis is maintained predominantly through the regulation of renal excretion. Where renal excretion of $K^+$ is impaired, elevated serum $K^+$ levels will occur. Hyperkalemia is a condition wherein serum potassium is greater than about 5.0 mEq/L.

While mild hyperkalemia, defined as serum potassium of about 5.0-6mEq/L, is not normally life threatening, moderate to severe hyperkalemia (with serum potassium greater than about 6.1 mEq/L) can have grave consequences. Cardiac arrythmias and altered ECG waveforms are diagnostic of hyperkalemia. See Schwartz, MW (1987) Am J Nurs 87: 1292-1299. When serum potassium levels increases above about 9mEq/L, atrioventricular dissociation, ventricular tachycardia, or ventricular fibrillation can occur.

Hyperkalemia is rare in the general population of healthy individuals. However, certain groups definitely exhibit a higher incidence of hyperkalemia. In patients who are hospitalized, the incidence of hyperkalemia ranges from about 1-10%, depending on the definition of hyperkalemia. Patients at the extremes of life, either premature or elderly, are at high risk. The presence of decreased renal function, genitourinary disease, cancer, severe diabetes, and polypharmacy can also predispose patients to hyperkalemia.

Most of the current treatment options for hyperkalemia are limited to use in hospitals. For example, exchange resins, such as Kayexalate, are not suitable for outpatient or chronic treatment, due to the large doses necessary that leads to very low patient compliance, severe GI side effects and significant introduction of sodium (potentially causing hypernatremia and related fluid retention and hypertension). Diuretics that can remove sodium and potassium from patients via the kidneys are often limited in their efficacy due to underlying kidney disease and frequently related diuretic resistance. Diuretics are also contraindicated in patients where a drop in blood pressure and volume depletion are undesired (e.g. CHF patients that in addition to suffering from low blood pressure are often on a combination of drugs such as ACE inhibitors and potassium sparing diuretics such as spironolactone that can induce hyperkalemia).

Kim Ho-Jung et al.: "Therapeutic Approach to Hyperkalemia" 1 October 2002 (2002-10-01); Nephron, S. Karger AG, Switzerland LNKD-DOI: 10.1159/000005375, Page(s) 33-40, ISSN: 0028-2766 discloses a method for treating hyperkalemia including insulin as an active ingredient.

Overall, it would be desirable to obtain higher binding capacity materials for the treatment of hyperkalemia, such materials preferably having a greater binding in the physiological pH range for potassium, which are also non-degradable, non-absorbable and have decreased toxic effects.

### SUMMARY OF THE INVENTION

The present invention provides compositions and methods for the removal of potassium ions from the gastro-intestinal tract, according to claims 1 and 16.

The potassium binding polymer is preferably a poly-fluoroacrylic acid polymer, a poly-difluoromaleic acid polymer, poly-sulfonic acid, or a combination thereof. In other embodiments the polymer comprises 2-fluoroacrylic acid crosslinked with divinylbenzene, ethylene bisacrylamide, N,N'-bis(vinylsulfonylacetyl) ethylene diamine, 1,3-bis(vinylsulfonyl) 2-propanol, vinylsulfone, N,N'-methylenebisacrylamide polyvinyl ether, polyallylether, or a combination thereof.

In a preferred embodiment, the potassium binding polymer is a $\alpha$-fluoroacrylate polymer crosslinked with divinyl benzene.

The compositions described herein are suitable for therapeutic and/or prophylactic use in the treatment of hyperkalemia. Potassium binding compositions may be used in combination with drugs that cause potassium retention such as potassium-sparing diuretics, angiotensin-converting enzyme inhibitors (ACEIs), Angiotensin receptor blockers (ARBs), non-steroidal anti-inflammatory drugs, heparin, or trimethoprim.

### BRIEF DESCRIPTION OF THE DRAWINGS

Figure 1 depicts starting cation concentrations in a meal mimic.

Figure 2 depicts binding of cations by resins in a meal mimic.

Figure 3 depicts the original concentrations of cations in the feces of two subjects.

Figure 4 depicts the binding of cations in human fecal extracts to cation exchange resins.

Figure 5 depicts the membrane preparation for determination of ion permeability.

Figure 6 depicts the binding data of different polyethyleneimine coated beads for different cations.

Figure 7 depicts the effect of a Eudragit RL 100 shell on magnesium and potassium binding.

Figure 8 depicts binding of magnesium on benzylated polyethyleneimine coated Dowex (K) beads.

Figure 9 depicts the stability of Ben(84)-PEI coated Dowex (K) beads under acid conditions representative of the acidic conditions in the stomach.

Figure 10 depicts potassium and magnesium binding by Dowex beads coated with benzylated polyethyleneimine.

Figure 11 depicts magnesium binding by fluoroacrylic acid beads with benzylated polyethylene imine shell.

Figure 12 depicts a setup for determining membrane permeability.

Figure 13 depicts the permeability of benzylated polyethyleneimine membrane.

Figure 14 depicts the permeability and permselectivity of membranes comprising of mixtures of Eudragit RL100 and Eudragit RS 100.

Figure 15 depicts the effects of bile acids on potassium binding by Dowex(Li) coated with polyethyleneimine.

Figure 16 depicts the effect of pH on $\alpha$-fluoroacrylate -acrylic acid copolymer.

Figure 17 depicts levels of excretion of cations in rats following administration of fluoroacrylate polymer and Kayexalate.

## DETAILED DESCRIPTION OF THE INVENTION

The present invention provides methods, polymeric pharmaceutical compositions for the treatment of animal subjects. The terms "animal subject" and "animal" as used herein includes humans as well as other mammals. In particular, the present invention provides polymeric compositions for the removal of potassium ions. Preferably, these compositions are used for the removal of potassium ions from the gastrointestinal tract of animal subjects.

One aspect of the invention is a method of removing potassium ions with a potassium-binding polymeric composition. In one embodiment, the potassium-binding polymeric composition has high capacity and/or selectivity for binding potassium and does not significantly release the bound potassium in the gastrointestinal tract. It is preferred that the polymeric composition exhibit selective binding for potassium ions.

It is preferred that the polymeric compositions of the present invention exhibit high capacity and/or selectivity for potassium ions. The term "high capacity" as used herein encompasses an average *in vivo* binding of about 1.5 mmol or more of potassium per gm of polymer. Typically, this in *vivo* binding capacity is determined in a human. Techniques for determining *in vivo* potassium binding capacity in a human are well known in the art. For example, following administration of a potassium-binding polymer to a patient, the amount of potassium in the feces can be used to calculate the in *vivo* potassium binding capacity. The average *in vivo* binding is preferably calculated in a set of normal human subjects, this set being about 5 human subjects, preferably about 10 human subjects, even more preferably about 25 human subjects, and most preferably about 50 human subjects.

The average *in vivo* potassium binding capacity can be equal to or more than about 1.5 mmol per gm of polymer in a human. The *in vivo* potassium binding capacity in a human is about 2 mmol or more per gm, about 3 mmol or more per gm, about 4 mmol or more per gm, or about 6 mmol or more per gm. The average *in vivo* potassium binding capacity in a human is about 2 mmol to about 6 mmol per gm in a human.

The capacity of the potassium binding polymers can also be determined *in vitro.* It is preferred that the *in vitro* potassium binding capacity is determined in conditions that mimic the physiological conditions of the gastro-intestinal tract, in particular the colon. The *in vitro* potassium binding capacity may be determined in solutions with a pH of about 5.5 or more. The *in vitro* potassium binding capacity in a pH of about 5.5 or more may be equal to or more than 6 mmol per gm of polymer. A range of *in vitro* potassium binding capacity in a pH of about 5.5 or more is about 6 mmol to about 12 mmol per gm of polymer. The *in vitro* potassium binding capacity in a pH of about 5.5 or more may be equal to about 6 mmol or more per gm, about 8 mmol or more per gm, about 10 mmol or more per gm, or about 12 mmol or more per gm.

The higher capacity of the polymeric composition enables the administration of a lower dose of the composition. Typically the dose of the polymeric composition used to obtain the desired therapeutic and/or prophylactic benefits is about 0.5 gm/day to about 25 gm/day. Most preferred is about 15 gm/day or less. A preferred dose range is about 5 gm/day to about 20 gm/day, more preferred is about 5 gm/day to about 15 gm/day, even more preferred is about 10 gm/day to about 20 gm/day, and most preferred is about 10 gm/day to about 15 gm/day. Preferably the dose is administered about three times a day with meals, most preferably the dose is administered once a day.

It is also preferred that the compositions described herein retain a significant amount of the bound potassium. Preferably, the potassium is bound by the polymer in the colon and not released prior to excretion of the polymer in the feces. The term "significant amount" as used herein is not intended to mean that the entire amount of the bound potassium is retained. It is preferred that at least some of the bound potassium is retained, such that a therapeutic and/or prophylactic

benefit is obtained. Preferred amounts of bound potassium that can be retained range from about 5% to about 100%. It is preferred that the polymeric compositions retain about 25% of the bound potassium, more preferred is about 50%, even more preferred is about 75% and most preferred is retention of about 100% of the bound potassium. The period of retention is preferred to be during the time that the composition is being used therapeutically and/or prophylactically, In the embodiment in which the composition is used to bind and remove potassium from the gastrointestinal tract, the retention period is the time of residence of the composition in the gastro-intestinal tract and more particularly the average residence time in the colon.

Preferably the potassium binding polymers are not absorbed from the gastro-intestinal tract. The term "non-absorbed" and its grammatical equivalents is not intended to mean that the entire amount of administered polymer is not absorbed. It is expected that certain amounts of the polymer may be absorbed. It is preferred that about 90% or more of the polymer is not absorbed, preferably about 95% or more is not absorbed, even more preferably about 97% or more is not absorbed, and most preferably about 98% or more of the polymer is not absorbed.

**Potassium-Binding Polymers**

The potassium-binding polymers comprise acid groups in their protonated or ionized form, such as sulfonic ($-SO_3^-$), sulfuric ($-OSO_3^-$), carboxylic ($-CO_2^-$), phosphonic ($-PO_3^{--}$), phosphoric ($-(OPO_3^{--})$), or sulfamate ($-NHSO_3^-$). Preferably, the fraction of ionization of the acid groups is greater than about 75% at the physiological pH in the colon and the potassium binding capacity is greater than about 5 mmol/gm. Preferably the ionization of the acid groups is greater than about 80%, more preferably it is greater than about 90%, and most preferably it is about 100%. In certain embodiments the acid containing polymers contain more than one type of acid groups. In certain embodiments the acid containing polymers are administered in their anhydride form and generate the ionized form when contacted with physiological fluids.

A $pK_a$-decreasing group, an electron-withdrawing substituent, is located adjacent to the acid group, preferably it is located in the alpha or beta position of the acid group. The electron-withdrawing substituents are a hydroxyl group, an ether group, an ester group, or an halide atom, and most preferably F. Preferred acid groups are sulfonic ($-SO_3^-$), sulfuric ($-OSO_3^-$), carboxylic ($-CO_2^-$), phosphonic ($-PO_3^{--}$), phosphoric ($-(OPO_3^{--})$), or sulfamate ($-NHSO_3^-$). Other preferred polymers result from the polymerization of alpha-fluoro acrylic acid, difluoromaleic acid, or an anhydride thereof.

Examples of other suitable monomers for potassium-binding polymers are included in Table 1.

TABLE 1: Examples of cation exchange moieties - structures & theoretical binding capacities

| | Molar mass per charge | Theoretical capacity | Fraction of titrable H @pH 3 | Fraction of titrable H @ pH 6 | Expected Capacity @pH3 | Expected Capacity @pH6 |
|---|---|---|---|---|---|---|
| 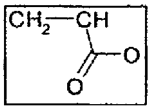 | 71 | 14.1 | 0.05 | .35 | 0.70 | 4.93 |
| 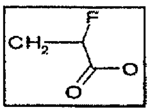 | 87 | 11.49 | 0.2 | 0.95 | 2.3 | 10.92 |
| 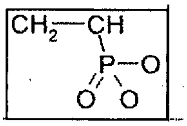 | 53 | 18.9 | 0.25 | 0.5 | 4.72 | 9.43 |
| 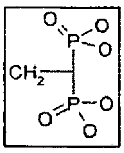 | 47.5 | 21.1 | 0.25 | 0.5 | 5.26 | 10.53 |
| 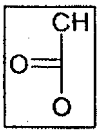 | 57 | 17.5 | 0.1 | 0.5 | 1.75 | 8.77 |

(continued)

| | Molar mass per charge | Theoretical capacity | Fraction of titrable H @pH 3 | Fraction of titrable H @ pH 6 | Expected Capacity @pH3 | Expected Capacity @pH6 |
|---|---|---|---|---|---|---|
| 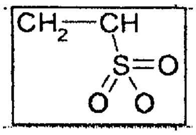 | 107 | 9.3 | 1 | 1 | 9.35 | 9.35 |
| 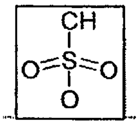 | 93 | 10.8 | 1 | 1 | 10.75 | 10.75 |
| 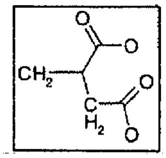 | 63 | 15.9 | 0 | 0.4 | 0 | 6.35 |
| 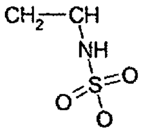 | 125 | 8 | 1 | 1 | 8 | 8 |
| 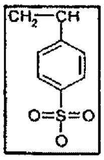 | 183 | 5.5 | 1 | 1 | 5.46 | 5.46 |
| 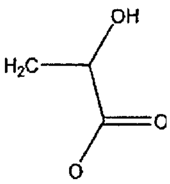 | 87 | 11.49 | .1 | .6 | 1.14 | 6.89 |

Other suitable cation exchange moieties include:

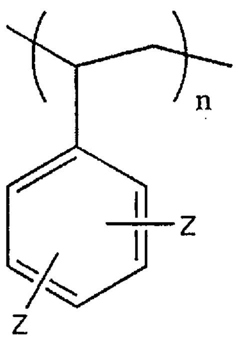

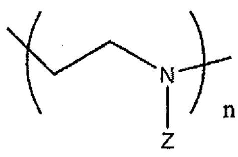
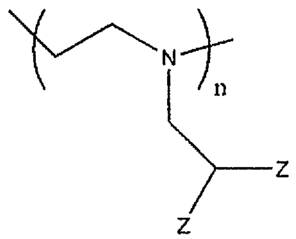

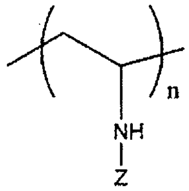
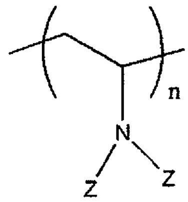
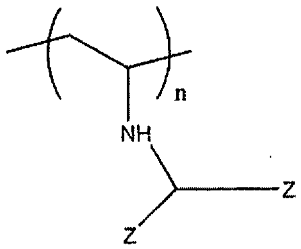
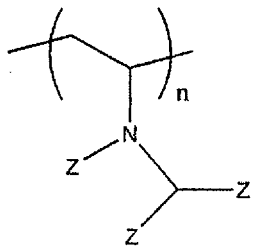

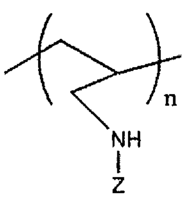
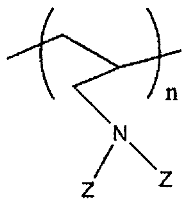
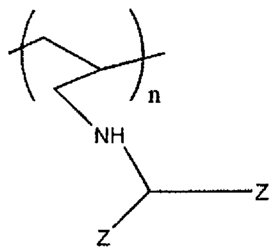
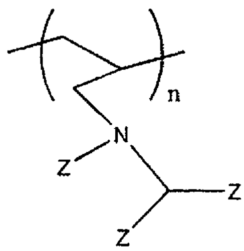

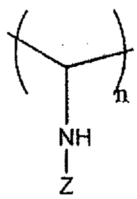
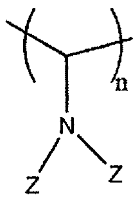
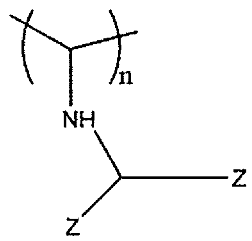
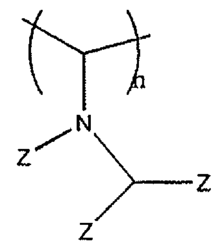

wherein n is equal to or greater than one and Z represents either $SO_3H$ or $PO_3H$. Preferably n is about 50 or more, more preferably n is about 100 or more, even more preferred is n about 200 or more, and most preferred is n about 500 or more.

Suitable phosphonate monomers include vinyl phosphonate, vinyl 1,1 bis phosphonate, and ethylenic derivatives of phosphonocarboxylate esters, oligo(methylenephosphonates), and hydroxyethane-1,1-diphosphonic acid. Methods of synthesis of these monomers are well known in the art.

Sulfamic (i.e. when Z=$SO_3H$) or phosphoramidic (i.e. when Z= $PO_3H$) polymers can be obtained from amine polymers or monomer precursors treated with a sulfonating agent such as sulfur trioxide/amine adducts or a phosphonating agent such as $P_2O_5$, respectively. Typically, the acidic protons of phosphonic groups are exchangeable with cations, like sodium or potassium, at pH of about 6 to about 7.

Free radical polymers derived from monomers such as vinyl sulfonate, vinylphosphonate, or vinylsulfamate can also be used.

Preferred monomers for use herein are α-fluoroacrylate and difluoromaleic acid, α -fluoroacrylate being most preferred. This monomer can be prepared from a variety of routes, see for example, Gassen et al, J. Fluorine Chemistry, 55, (1991) 149-162, KF Pittman, C. U., M. Ueda, et al. (1980). Macromolecules 13(5): 1031-1036. Difluoromaleic acid is preferred by oxidation of fluoroaromatic compounds (Bogachev et al, Zhurnal Organisheskoi Khimii, 1986, 22(12), 2578-83), or fluorinated furans derivatives (See U.S. patent 5,112,993). A preferred mode of synthesis of α -fluoroacrylate is given in EP 415214.

Other methods comprise the step-growth polymerization from phosphonate, carboxylic, phosphate, sulfinate, sulfate and sulfonate functionals compounds. High density polyphosphonates such as Briquest, marketed by Rhodia,

are particularly useful.

Polymers may include ion exchange resins synthesized from naturally occurring polymers, such as saccharide polymers and semi-synthetic polymers, optionally functionalized to create ion exchange sites on the backbone or on the pendant residues. Examples of polysaccharides of interest include materials from vegetal or animal origins, such as cellulosic materials, hemicellulose, alkyl cellulose, hydroxyalkyl cellulose, carboxymethylcellulose, sulfoethylcellulose, starch, xylan, amylopectine, chondroitin, hyarulonate, heparin, guar, xanthan, mannan, galactomannan, chitin and chitosan. Polymers that do not degrade under the physiological conditions of the gastrointestinal tract and remain non-absorbed are carboxymethylcellulose, chitosan, and sulfoethylcellulose.

The potassium binding polymer can be encased in a dialysis bag, paper bag, microporous matrix, polymer gel, hollow fibers, vesicles, capsules, tablet, or a film.

The polymers can be formed by polymerization processes using heterogeneous mode. The particles are prepared by polymerization of a monomer in an emulsion, suspension, miniemulsion or dispersion process. The continuous phase is either an aqueous vehicle or an organic solvent. When a suspension process is used, any suitable type of variants is possible, including methods such as "templated polymerization," "multistage'seeded suspension," all of which yielding mostly monodisperse particles. In one particular embodiment, the beads are formed using a "jetting" process (see U.S. patent 4,427,794), whereby a "tube of liquid containing a monomer plus initiator mixture is forced through a vibrating nozzle into a continuous phase. The nozzles can be arranged in spinning turret so as to force the liquid under centrifugal force.

A preferred process to produce alpha-fluoroacrylate beads is direct suspension polymerization. Typically, suspension stabilizers, such as polyvinyl alcohol, are used to prevent coalescence of particles during the process. It has been observed that the addition of NaCl in the aqueous phase decreased coalescence and particle aggregation. Other suitable salts for this purpose include salts that solubilize in the aqueous phase. In this embodiment, water soluble salts are added at a weight % comprised between about 0.1 to about 10, preferably comprised between about 2 to about 5 and even more preferably between about 3 and about 4.

It has been observed that in the case of alpha-fluoroacrylate esters (e.g. MeFA) suspension polymerization, the nature of the free radical initiator plays a role in the quality of the suspension in terms of particle stability, yield of beads, and the conservation of a spherical shape. Use of water-insoluble free radical initiators, such as lauryl peroxide, led to the quasi absence of gel and produced beads in a high yield. It was found that free radical initiators with water solubility lower than 0.1 g/L preferably lower than 0.01 g/L led to optimal results. In preferred embodiments, polyMeFA beads are produced with a combination of a low water solubility free radical initiator and the presence of salt in the aqueous phase, such as NaCl.

In some embodiments wherein the potassium binding polymer is used without a shell, the potassium binding polymer is not Kayexalate, sodium polystyrene sulfonate, or an ammonium form of polystyrene sulfonate.

Crown ethers and crown-ether like molecules are also disclosed as potassium binding polymers. Crown ethers show selectivity for certain alkali metals over others, based on the hole-size and the size of the metal ion. See Tables 2, 3 and 4 and Pedersen, C.J. 1987. Charles J. Pederson - Nobel Lecture. The discovery of crown ethers. In Nobel Lectures, Chemistry 1981-1990. T. Frangsmyr, editor. World Scientific Publishing Co., Singapore.

Crown ethers are also disclosed as shell materials to decrease the passage of sodium, magnesium, calcium and other interfering molecules to the core and as a result, increase the *in vivo* binding capacity of a core polymer.

***Table 2: Diameters of holes in Sample Crown Ethers, in Angstrom unites***

| Macrocyclic Polyethers | Diameters |
|---|---|
| All 14-crown-4 | 1.2-1.5 |
| All 15-crown-5 | 1.7-2.2 |
| All 18-crown-6 | 2.6-3.2 |
| All 21-crown-7 | 3.4-4.3 |

Table 3: Complexable cations and their diameters in Angstrom units

| Group I | | Group II | | Group III | | Group IV | |
|---|---|---|---|---|---|---|---|
| Li | 1.36 | | | | | | |
| Na | 1.94 | | | | | | |
| K | 2.66 | Ca | 1.98 | | | | |
| Cu(I) | 1.92 | Zn | 1.48 | | | | |
| Rb | 2.94 | Sr | 2.26 | | | | |

(continued)

| Group I | | Group II | | Group III | | Group IV | |
|---|---|---|---|---|---|---|---|
| Ag | 2.52 | Cd | 1.94 | | | | |
| Cs | 3.34 | Ba | 2.68 | La | 2.30 | | |
| Au(I) | 2.88 | Hg(II) | 2.20 | Ti(I) | 2.80 | Pb(II) | 2.40 |
| Fr | 3.52 | Ra | 2.80 | | | | |
| $NH_4$ | 2.86 | | | | | | |

Table 4: Relative binding of sample alkali metal ions by sample crown ethers

| Polyether | $Li^+$ | $Na^+$ | $K^+$ | $Cs^+$ |
|---|---|---|---|---|
| Dicyclohexyl-14-crown-4 | 1.1 | 0 | 0 | 0 |
| Cyclohexl-15-crown-5- | 1.6 | 19.7 | 8.7 | 4.0 |
| Dibenzo-18-crown-6 | 0 | 1.6 | 25.2 | 5.8 |
| Dicyclohexyl-18-crown-6 | 3.3 | 25.6 | 77.8 | 44.2 |
| DIcyclohexyl-21-crown-7 | 3.1 | 22.6 | 51.3 | 49.7 |
| Dicyclohexyl-24-crown-8 | 2.9 | 8.9 | 20.1 | 18.1 |

The potassium binding polymers typically include cationic counterions. The cations can be metallic, non-metallic, or a combination thereof. Examples of metallic ions include, but are not limited to, $Ca^2$+-form, $H^+$-form, $NH^{4+}$-form, $Na^+$-form, or a combination thereof. Examples of non-metallic ions include, but are not limited to, alkylammonium, hydroxyalkylammonium, choline, taurine,carnitine, guanidine, creatine, adenine, and aminoacids or derivatives thereof.

In preferred embodiments, the potassium binding polymers described herein have a decreased tendency to cause side-effects such as hypernatremia and acidosis due to the release of detrimental ions. The term "detrimental ions" is used herein to refer to ions that are not desired to be released into the body by the compositions described herein during their period of use. Typically, the detrimental ions for a composition depend on the condition being treated, the chemical properties, and/or binding properties of the composition. For example, the detrimental ion could be $H^+$ which can cause acidosis or $Na^+$ which can cause hypernatremia. Preferably the ratio of potassium bound to detrimental cations introduced is 1: about 2.5 to about 4.

**Methods of Treatments**

The methods and compositions described herein are suitable for treatment of hyperkalemia caused by disease and/or use of certain drugs.

In some embodiments of the invention, the compositions and methods described herein are used in the treatment of hyperkalemia caused by decreased excretion of potassium, especially when intake is not reduced. A common cause of decreased renal potassium excretion is renal failure (especially with decreased glomerular filtration rate), often coupled with the ingestion of drugs that interfere with potassium excretion, e.g., potassium-sparing diuretics, angiotensin-converting enzyme inhibitors (ACEIs), non-steroidal anti-inflammatory drugs, heparin, or trimethoprim. Impaired responsiveness of the distal tubule to aldosterone, for example in type IV renal tubular acidosis observed with diabetes mellitus as well as sickle cell disease and/or chronic partial urinary tract obstruction Is another cause of reduced potassium secretion. Secretion is also inhibited in diffuse adrenocortical insufficiency or Addison's disease and selective hypoaldosteronism. Hyperkalemia is common when diabetics develop hypoteninemic hypoaldosteronism or renal insufficiency (Mandal, A.K. 1997. Hypokalemia and hyperkalemia. Med Clin North Am. 81:611-39).

In certain preferred embodiments, the potassium binding polymers described herein are administered chronically. Typically, such chronic treatments will enable patients to continue using drugs that cause hyperkalemia, such as potassium-sparing diuretics, ACEI's, non-steroidal anti-inflammatory drugs, heparin, or trimethoprim. Also, use of the polymeric compositions described herein will enable certain patient populations, who were unable to use hyperkalemia causing drugs, to use such drugs.

In certain chronic use situations, the preferred potassium binding polymers used are those that are capable of removing less than about 5 mmol of potassium per day or in the range of about 5 - about 10 mmol of potassium per day. In acute conditions, it is preferred that the potassium binding polymers used are capable of removing about 15 - about 60 mmol of potassium per day.

In certain other embodiments, the compositions and methods described herein are used in the treatment of hyperkalemia caused by a shift from intracellular to extracellular space. Infection or trauma resulting in cell disruption,

especially rhabdomyolysis or lysis of muscle cells (a major potassium store), and tumor lysis can result in acute hyperkalemia. More often, mild-to-moderate impairment of intracellular shifting of potassium occurs with diabetic ketoacidosis, acute acidosis, infusion of argentine or lysine chloride for the treatment of metabolic alkalosis, or infusion of hypertonic solutions such as 50% dextrose or mannitol. □-receptor blocking drugs can cause hyperkalemia by inhibiting the effect of epinephrine.

In certain other embodiments, the compositions and methods described herein are used in the treatment of hyperkalemia caused by excessive intake of potassium. Excessive potassium intake alone is an uncommon cause of hyperkalemia. Most often, hyperkalemia is caused by indiscriminate potassium consumption in a patient with impaired mechanisms for the intracellular shift of potassium or renal potassium excretion. For example, sudden death among dialyzed patients who are noncompliant in diet can be attributed to hyperkalemia.

In the present invention, the potassium-binding polymers can be co-administered with other active pharmaceutical agents. This co-administration can include simultaneous administration of the two agents in the same dosage form, simultaneous administration in separate dosage forms, and separate administration. For example, for the treatment of hyperkalemia, the potassium-binding polymers and the core-shell compositions can be co-administered with drugs that cause the hyperkalemia, such as potassium-sparing diuretics, angiotensin-convening enzyme inhibitors, non-steroidal anti-inflammatory drugs, heparin, or trimethoprim. The drug being co-administered can be formulated together in the same dosage form and administered simultaneously. Alternatively, they can be simultaneously administered, wherein both the agents are present in separate formulations. In another alternative, the drugs are administered separately. In the separate administration protocol, the drugs may be administered a few minutes apart, or a few hours apart, or a few days apart.

The term "treating" as used herein includes achieving a therapeutic benefit and/or a prophylactic benefit. By therapeutic benefit is meant eradication, amelioration, or prevention of the underlying disorder being treated. For example, in a hyperkalemia patient, therapeutic benefit includes eradication or amelioration of the underlying hyperkalemia. Also, a therapeutic benefit is achieved with the eradication, amelioration, or prevention of one or more of the physiological symptoms associated with the underlying disorder such that an improvement is observed in the patient, notwithstanding that the patient may still be afflicted with the underlying disorder. For example, administration of a potassium-binding polymer to a patient suffering from hyperkalemia provides therapeutic benefit not only when the patient's serum potassium level is decreased, but also when an improvement is observed in the patient with respect to other disorders that accompany hyperpkalemia like renal failure. For prophylactic benefit, the potassium-binding polymers may be administered to a patient at risk of developing hyperpkalemia or to a patient reporting one or more of the physiological symptoms of hyperpkalemia, even though a diagnosis of hyperpkalemia may not have been made.

The pharmaceutical compositions of the present invention include compositions wherein the potassium binding polymers are present in an effective amount, i.e., in an amount effective to achieve therapeutic or prophylactic benefit. The actual amount effective for a particular application will depend on the patient (e.g., age, weight, etc.), the condition being treated, and the route of administration. Determination of an effective amount is well within the capabilities of those skilled in the art, especially in light of the disclosure herein.

The effective amount for use in humans can be determined from animal models. For example, a dose for humans can be formulated to achieve gastrointestinal concentrations that have been found to be effective in animals.

The dosages of the potassium binding polymers in animals will depend on the disease being, treated, the route of administration, and the physical characteristics of the patient being treated. Dosage levels of the potassium binding polymers for therapeutic and/or prophylactic uses can be from about about 0.5 gm/day to about 30 gm/day. It is preferred that these polymers are administered along with meals. The compositions may be administered one time a day, two times a day, or three times a day. Most preferred dose is about 15 gm/day or less. A preferred dose range is about 5 gm/day to about 20 gm/day, more preferred is about 5 gm/day to about 15 gm/day, even more preferred is about 10 gm/day to about 20 gm/day, and most preferred is about 10 gm/day to about 15 gm/day.

The compositions described herein can be used as food products and/or food additives. They can be added to foods prior to consumption or while packaging to decrease levels of potassium. The compositions can also be used in fodder for animals to lower $K^+$ levels, which is for example desirable for example in fodders for pigs and poultry to lower the water secretion.

**Formulations and Routes of Administration**

The polymeric compositions described herein or pharmaceutically acceptable salts thereof, can be delivered to the patient using a wide variety of routes or modes of administration. The most preferred routes for administration are oral, intestinal, or rectal.

If necessary, the polymers may be administered in combination with other therapeutic agents. The choice of therapeutic agents that can be co-administered with the compounds of the invention will depend, in part, on the condition being treated.

The polymers (or pharmaceutically acceptable salts thereof) may be administered per se or in the form of a pharmaceutical composition wherein the active compound(s) is in admixture or mixture with one or more pharmaceutically acceptable carriers, excipients or diluents. Pharmaceutical compositions for use in accordance with the present invention may be formulated in conventional manner using one or more physiologically acceptable carriers compromising excipients and auxiliaries which facilitate processing of the active compounds into preparations which can be used pharmaceutically. Proper formulation is dependent upon the route of administration chosen.

For oral administration, the compounds can be formulated readily by combining the active compound(s) with pharmaceutically acceptable carriers well known in the art. Such carriers enable the compounds of the invention to be formulated as tablets, pills, dragees, capsules, liquids, gels, syrups, slurries, suspensions, wafers, and the like, for oral ingestion by a patient to be treated. In one embodiment, the oral formulation does not have an enteric coating. Pharmaceutical preparations for oral use can be obtained as a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, mehtyl cellulose, hydroxypropylmethyl-cellulose, sodium carboxymethylcellulose, and/or polyvinyl pyrrolidone (PVP). If desired, disintegrating agents may be added, such as the cross-linked polyvinyl pyrrolidone, agar, or alginic acid or a salt thereof such as sodium alginate.

Dragee cores can be provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

For administration orally, the compounds may be formulated as a sustained release preparation. Numerous techniques for formulating sustained release preparations are known in the art.

Pharmaceutical preparations which can be used orally include push-fit capsules made of gelatin, as well as soft, seated capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for administration.

In some embodiments the polymers of the invention are provided as pharmaceutical compositions in the form of chewable tablets. In addition to the active ingredient, the following types of excipients are commonly used: a sweetening agent to provide the necessary palatability, plus a binder where the former is inadequate in providing sufficient tablet hardness; a lubricant to minimize frictional effects at the die wall and facilitate tablet ejection; and, in some formulations a small amount of a disintegrant is added to facilitate mastication. In general excipient levels in currently-available chewable tablets are on the order of 3-5 fold of active ingredient(s) whereas sweetening agents make up the bulk of the inactive ingredients.

The present invention provides chewable tablets that contain a polymer or polymers of the invention and one or more pharmaceutical excipients suitable for formulation of a chewable tablet. The polymer used in chewable tablets of the invention preferably has a swelling ratio while transiting the oral cavity and in the esophagus of less than about 5, preferably less than about 4, more preferably less than about 3, more preferably less than 2.5, and most preferably less than about 2. The tablet comprising the polymer, combined with suitable excipients, provides acceptable organoleptic properties such as mouthfeel, taste, and tooth packing, and at the same time does not pose a risk to obstruct the esophagus after chewing and contact with saliva.

In some aspects of the invention, the polymer(s) provide mechanical and thermal properties that are usually performed by excipients, thus decreasing the amount of such excipients required for the formulation. In some embodiments the active ingredient (e.g., polymer) constitutes over about 30%, more preferably over about 40%, even more preferably over about 50%, and most preferably more than about 60% by weight of the chewable tablet, the remainder comprising suitable excipient(s). In some embodiments the polymer comprises about 0.6 gm to about 2.0 gm of the total weight of the tablet, preferably about 0.8 gm to about 1.6 gm. In some embodiments the polymer comprises more than about 0.8 gm of the tablet, preferably more than about 1.2 gm of the tablet, and most preferably more than about 1.6 gm of the tablet. The polymer is produced to have appropriate strength/friability and particle size to provide the same qualities for which excipients are often used, e.g., proper hardness, good mouth feel, compressibility, and the like. Unswelled particle size for polymers used in chewable tablets of the invention is less than about 80, 70, 60, 50, 40, 30, or 20 microns mean diameter. In preferred embodiments, the unswelled particle size is less than about 80, more preferably less than about 60, and most preferably less than about 40 microns.

Pharmaceutical excipients useful in the chewable tablets of the inventon include a binder, such as microcrystalline cellulose, colloidal silica and combinations thereof (Prosolv 90), carbopol, providone and xanthan gum; a flavoring

agent, such as sucrose, mannitol, xylitol, maltodextrin, fructose, or sorbitol; a lubricant, such as magnesium stearate, stearic acid, sodium stearyl fumurate and vegetable based fatty acids: and, optionally, a disintegrant, such as oroscarmellose sodium, gellan gum, low-substituted hydroxypropyl ether of cellulose, sodium starch glycolate. Other additives may include plasticizers, pigments, talc, and the like. Such additives and other suitable ingredients are well-known in the art; see, e.g., Gennaro AR (ed), Remington's Pharmaceutical Sciences, 20th Edition.

In some embodiments the invention provides a pharmaceutical composition formulated as a chewable tablet, comprising a polymer described herein and a suitable excipient. In some embodiments the Invention provides a pharmaceutical composition formulated as a chewable tablet, comprising a polymer described herein, a filer, and a lubricant. In some embodiments the invention provides a pharmaceutical composition formulated as a chewable tablet, comprising a polymer described herein, a filler, and a lubricant, wherein the filler is chosen from the group consisting of sucrose, mannitol, xylitol, maltodextrin, fructose, and sorbitol, and wherein the lubricant is a magnesium fatty acid salt, such as magnesium stearate.

The tablet may be of any size and shape compatible with chewability and mouth disintegration, preferably of a cylindrical shape, with a diameter of about 10 mm to about 40 mm and a height of about 2 mm to about 10 mm, most preferably a diameter of about 22 mm and a height of about 6 mm.

In one embodiment, the polymer is pre-formulated with a high Tg / high melting point low molecular weight excipient such as mannitol, sorbose, sucrose in order to form a solid solution wherein the polymer and the excipient are intimately mixed. Method of mixing such as extrusion, spray-drying, chill drying, lyophilization, or wet granulation are useful. Indication of the level of mixing is given by known physical methods such as differential scanning calorimetry or dynamic mechanical analysis.

Methods of making chewable tablets containing pharmaceutical ingredients, including polymers, are known in the art. See, e.g., European Patent Application No. EP373852A2 and U.S. Patent No. 6,475.610, and Remington's Pharmaceutical Sciences.

In some embodiments the polymers of the invention are provided as pharmaceutical compositions in the form of liquid formulations, In some embodiments the pharmaceutical composition contains an ion-binding polymer dispersed in a suitable liquid excipient. Suitable liquid excipients are known in the art; see, e.g., *Remington's Pharmaceutical Sciences*.

## EXAMPLES

### Example 1: Preparation of polymers with high binding capacity

***Materials:***

All chemicals were purchased from commercial sources and used as received. All reactions were carried out under nitrogen. Chemical structures and used abbreviations are given below in Tables 6 and 7.

Table 6: Monomer Abbreviations and Structures

| Abbreviation | Chemical name | Structure | Molecular Weight | CAS # |
|---|---|---|---|---|
| Na-VSA | vinylsulfonic acid sodium salt | 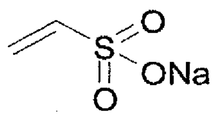 | 130.1 | 3039-83-6 |
| FAA | 2-fluoroacrylic acid or $\alpha$-fluoroacrylic acid or 2-fluoropropenoic acid | 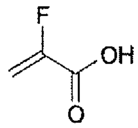 | 90.05 | 430-99-9 |
| VPA | vinylphosphonic acid | 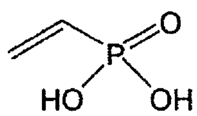 | 108.03 | 1746-03-8 |

Table 7: Crosslinker Abbreviations and Structures

| Abbreviation | Chemical name | Structure | Molecular Weight | CAS# |
|---|---|---|---|---|
| X-V-1 | ethylenebisacrylamide | 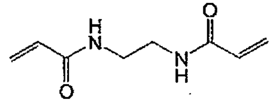 | 168.2 | 2956-58-3 |
| X-V-2 | | 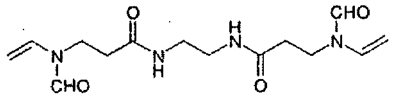 | 310.36 | |
| X-V-3 | | 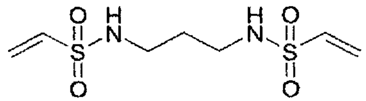 | 254.33 | |
| X-V-4 | N,N'-bis(vinylsulfonylacetyl) ethylene diamine | 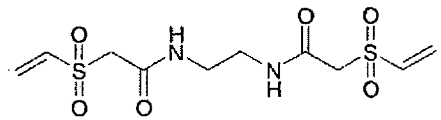 | 324.38 | 66710-66-5 |
| X-V-5 | 1,3-bis(vinylsulfonyl)2-propanol | 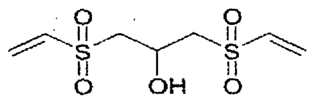 | 240.3 | 67006-32-0 |
| X-V-6 | vinylsulfone | 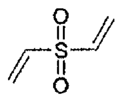 | 118.15 | 77-77-0 |
| X-V-7 | N,N'-methylenebisacrylamide | 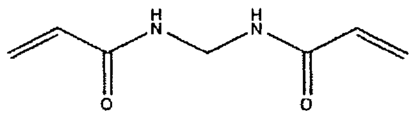 | 154.17 | 110-26-9 |
| ECH | epichlorohydrin | 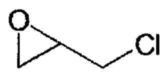 | 92.52 | |

Initiators: VA-044: 2,2'-azobis[2-(2-imidazolin-2-yl)propane] dihydrochloride; $K_2S_2O_8$, potassium persulfate

***General procedure for gel preparation from FAA:***

To a 15-ml test tube were charged FAA, X-V-1, and water, followed by a magnetic stirbar. The mixture was stirred at 45°C for 20 minutes and VA-044 (100mg/ml solution in water) was added. The solution gelled and was kept at 45°C for 4 hours, then cooled to room temperature.

The gel was transferred to a 50-ml polypropylene tube and water was added to a total volume of 30 ml. The gel was crushed with a spatula, and further milled with an Ultra-Turrax. The tube was capped and centrifuged at 3000 rpm for 30 minutes and the supernatant solution was decanted off. To the gel was added 1.0M HCl to a total volume of 45 ml and tube was capped and tumbled for 30 minutes. The tube was centrifuged at 3000 rpm for 30 minutes and supernatant solution was decanted off. The same tumbling-centrifuging procedure was repeated once with 1.0M HCl and three times with nanopure water. The gel was freeze-dried for three days. The reaction solution composition and gel yield are displayed in Table 8.

Table 8: Synthesis of FAA gels

| Sample # | Reaction solution composition | | | | Yield (mg) |
|---|---|---|---|---|---|
| | FAA (mg) | X-V-1 (mg) | Water (mL) | VA-044 (mL) | |
| 628A | 757 | 19 | 0.757 | 0.038 | 740 |
| 628B | 737 | 37 | 0.737 | 0.037 | 760 |
| 628C | 730 | 73 | 0.730 | 0.037 | 760 |
| 628D | 691 | 138 | 0.691 | 0.035 | 780 |

***General procedure for gel preparation from NaVSA:***

Commercially available NaVSA was converted into acid form and purified by vacuum distillation according to a method described by Breslow et al (J. Am. Chem. Soc., 1954, 76, 6399-6401). The pure acid was then dissolved in water and neutralized with NaOH solution carefully at 0°C. The colorless salt solution was concentrated by vacuum distillation to a concentration of 56 wt. %.

To a 15-ml test tube were charged NaVSA solution, crosslinker, and a magnetic stirbar and the mixture was stirred at 45°C for 20 minutes. VA-044 (50mg/mL solution in water) or $K_2S_2O_8$ (50mg/mL solution in water) was added. The solution was stirred at 45°C (if VA-044 used) or 50°C (if $K_2S_2O_8$ used) for 16 hours, then cooled to room temperature. The gel was purified according to the same procedure as used for FAA gel. The reaction solution composition and gel yield were displayed in Table 9.

Table 9: Synthesis of NaVSA gels

| Sample # | Reaction solution composition | | | | | Yield (mg) |
|---|---|---|---|---|---|---|
| | NaVSA (mL) | X-V-1 (mg) | X-V-5 (mg) | VA-044 (mL) | $K_2S_2O_8$ (mL) | |
| 100851A1 | 1.493 | 28 | 0 | 0.056 | 0 | 0 |
| 100851A2 | 1.493 | 56 | 0 | 0.056 | 0 | 400 |
| 100851A3 | 1.493 | 112 | 0 | 0.056 | 0 | 740 |
| 100851A4 | 1.493 | 225 | 0 | 0.056 | 0 | 590 |
| 100851B1 | 1.493 | 0 | 28 | 0.056 | 0 | 550 |
| 100851B2 | 1.493 | 0 | 56 | 0.056 | 0 | 830 |
| 100851B3 | 1.493 | 0 | 112 | 0.056 | 0 | 890 |
| 100851B4 | 1.493 | 0 | 225 | 0.056 | 0 | 800 |
| 100851C1 | 1.493 | 28 | 0 | 0 | 0.056 | 0 |
| 100851C2 | 1.493 | 56 | 0 | 0 | 0.056 | 420 |
| 100851C3 | 1.493 | 112 | 0 | 0 | 0.056 | 760 |
| 100851C4 | 1.493 | 225 | 0 | 0 | 0.056 | 730 |
| 100855D1 | 1.493 | 0 | 28 | 0 | 0.056 | 390 |
| 100851 D2 | 1.493 | 0 | 56 | 0 | 0.056 | 540 |
| 100851 D3 | 1.493 | 0 | 112 | 0 | 0.056 | 890 |

(continued)

| Sample # | Reaction solution composition | | | | | Yield (mg) |
|---|---|---|---|---|---|---|
| | NaVSA (mL) | X-V-1 (mg) | X-V-5 (mg) | VA-044 (mL) | $K_2S_2O_8$ (mL) | |
| 100851 D4 | 1.493 | 0 | 225 | 0 | 0.056 | 720 |

***General procedure for gel preparation from copolymerization of Na VSA and FAA:***

To a 15-ml test tube were charged FAA and NaVSA solution, followed by a magnetic stirbar. The mixture was stirred at room temperature for 10 minutes and all FAA dissolved. X-V-1 was added and mixture was stirred at room temperature for 10 minutes, then at 45°C for 20 minutes. VA-044 (100mg/ml solution in water) was added and the solution was stirred at 45°C for 3 hours, then cooled to room temperature. The gel was purified according to the same procedure as used for FAA gel. The reaction solution composition and gel yield were displayed in Table 10.

Table 10: Synthesis of NaVSA/FAA gels

| Sample # | Reaction solution composition | | | | Yield (mg) |
|---|---|---|---|---|---|
| | FAA (mg) | NaVSA (mL) | X-V-1 (mg) | Va-044 (mL) | |
| 101028A1 | 0 | 1.328 | 100 | 0.100 | 600 |
| 101028A2 | 100 | 1.195 | 100 | 0.100 | 630 |
| 101028A3 | 200 | 1.062 | 100 | 0.100 | 720 |
| 101028A4 | 300 | 0.930 | 100 | 0.100 | 780 |
| 101028A5 | 400 | 0.797 | 100 | 0.100 | 730 |
| 101028A6 | 500 | 0.664 | 100 | 0.100 | 700 |

***General procedure for gel preparation from copolymerization of AA and FAA:***

To a 15-ml test tube containing a magnetic stirbar, were charged FAA, X-V-1 and water, and the mixture was stirred until all solids dissolved. AA was added, followed by VA-044 (100mg/ml solution in water). The mixture was stirred at 45°C for 3 hours, then cooled to room temperature. The gel was purified according to the same procedure as used for FAA gel. The reaction solution composition and gel yield were displayed in Table 11.

Table 11: Synthesis of FAA/AA gels

| Sample # | Reaction solution composition | | | | | Yield (mg) |
|---|---|---|---|---|---|---|
| | FAA (mg) | AA (mL) | X-V-1 (mg) | Water (mL) | VA-044 (mL) | |
| 100982A1 | 800 | 0 | 80 | 0.764 | 0.040 | 770 |
| 100982A2 | 720 | 0.076 | 80 | 0.764 | 0.040 | 700 |
| 100982A3 | 640 | 0.152 | 80 | 0.764 | 0.040 | 730 |
| 100982A4 | 560 | 0.228 | 80 | 0.764 | 0.040 | 740 |
| 100982A5 | 480 | 0.304 | 80 | 0.764 | 0.040 | 740 |
| 100982A6 | 400 | 0.380 | 80 | 0.764 | 0.040 | 730 |

***General procedure for preparation of poly(vinylsulfamate) gel:***

Polyvinylamine hydrochloride (PVAm.HCl) was prepared according to a literature procedure by Badesso et al (in Hydrophilic Polymers: Performance with Environmental acceptance, P489-504). PVAm gel was prepared by the crosslinking reaction of PVAm.HCl with epichlorohydrin. The procedure was as follows: to a 100ml of round bottom flask was charged 33 wt% PVAm.HCl aqueous solution (15 gm, 62.9 mmol), followed by 50 wt% NaOH solution (2.63gm) to neutralize 50 mol% of PVAm.HCl. Epichlorohydrin (1.0 gm) was added and the mixture was stirred magnetically until stirring stopped due to gel formation. The gel was further cured at 65°C for 12 hours and transferred to a 50-ml polypropylene tube, and then water was added to a total volume of 30 ml. The gel was crushed with a spatula, and further milled with an Ultra-Turrax. The gel was washed with 1M HCl and nanopure water using the procedure described for FAA gel. Finally, PVAm gel was freeze dried for 3 days.

***General procedure for preparing poly(vinylsulfamate) gel:***

To a 20 ml vial was added 0.5 gm of PVAm gel and 10 ml of solvent. The mixture was heated at 60°C for 1 hour, then 0.5 gm of sulfur trioxide trimethylamine ($SO_3.N(CH_3)_3$) was added. Inorganic base, $Na_2CO_3$ or 2M NaOH solution, was added to the reaction mixture to maintain the pH above 9. The mixture was heated at 60°C for a certain time. The mixture was centrifuged, and supernatant solution was decanted off. The gel was washed with nanopure water until pH reached 7, and freeze dried. The reaction conditions and the conversion of amine group to sulfamate group are shown in Table 12.

Table 12: Preparation of poly(vinylsulfamate) gel

| Sample # | Ratio of $(CH_3)_3.SO_3$ to $NH_2$ | Base | Reaction time (hours) | Solvent | Conversion (%) |
|---|---|---|---|---|---|
| 001 | 1:1 | None | 3 | Water | 22.4 |
| 002 | 1:1 | None | 10 | Water | 37.1 |
| 003 | 1:1 | None | 22 | Water | 40.8 |
| 008 | 1:1.5 | $(CH_3)_3N$ | 22 | $(CH_3)_3N$ /water (20 vol%) | 65.5 |
| 010 | 1:1.5 | Pyridine | 22 | Pyridine/Water (20 wt%) | 4.84 |
| 013 | 1:1 | $Na_2CO_3$ | 22 | Water | 80.5 |
| 014 | 1:1.5 | $Na_2CO_3$ | 22 | Water | 86.1 |
| 015 | 1:1 | NaOH | 22 | Water | 72.5 |
| 016 | 1.5 | NaOH | 22 | water | 73.5 |

**Example 2: Binding Capacity Screening Protocol**

All experiments were performed in duplicate. Approximately 30mg of each polymer was aliquoted In duplicate into 16x100mm glass test tubes. Dowex 50W and Amberlite CG-50 were included in each experiment as internal controls. The relevant test binding buffer (Buffer 1, Buffer 2 or Buffer 3 below) was added to a final resin concentration of 2.5mg/ml. The test tubes were sealed using a Teflon membrane and incubated at room temperature, with constant end-over-end rotation, for at least one hour to allow the cations to achieve binding equilibrium with the polymers. The test tubes were then centrifuged at 500g for thirty minutes to isolate the resins. A sample of the supernatant was taken and the equilibrium concentrations of potassium ($K^+_{eq}$) and sodium ($Na^+_{eq}$) were determined by Ion Chromatography (IC). By comparing $K^+_{eq}$ and $Na^+_{eq}$ with the concentration of potassium in Buffer 1, Buffer 2 or Buffer 3 in the absence of polymer ($K^+_{start}$ and $Na^+_{start}$), the amount of cation (in mmoles cation/gram of polymer) was calculated. The ratio of sodium and potassium bound to the polymer was also calculated in this manner.

The capacity of each resin for Sodium and for Potassium was tested under some or all of the following conditions:

1. 75mM NaOH, 75mM KOH (pH not adjusted)
2. 50mM Citric Acid, 75mM KOH, 75mM NaOH, pH6.35 (with HCl)
3. 50mM Citric Acid, 75mM KOH, 75mM NaOH, pH 3 (with HCl)

TABLE 13: Binding capacities of phosphonic, carboxylic, and sulfonic polymers

| Sample Name | Description | Total mmoles ($Na^+$ +$K^+$) bound/gm resin, pH12.5 | $Na^+$:$K^+$ ratio at pH12.5 | Total mmoles ($Na^+$ + $K^+$) bound/gm resin, pH 6.25 | $Na^+$ :$K^+$ ratio at pH 6.25 | Total mmoles ($Na^+$ + $K^+$) bound/gm resin. pH3 | $Na^+$: $K^+$ratio at pH3 |
|---|---|---|---|---|---|---|---|
| 616B3 | NaVSA + 20 wt. % X-V-1 | | | | | | |
| 624B | NaVSA + 5 wt.% X-V-2 | | | | | | |

(continued)

| Sample Name | Description | Total mmoles ($Na^+$ +$K^+$) bound/gm resin, pH12.5 | $Na^+$:$K^+$ ratio at pH12.5 | Total mmoles ($Na^+$ + $K^+$) bound/gm resin, pH 6.25 | $Na^+$ :$K^+$ ratio at pH 6.25 | Total mmoles ($Na^+$ + $K^+$) bound/gm resin. pH3 | $Na^+$: $K^+$ratio at pH3 |
|---|---|---|---|---|---|---|---|
| 624C | NaVSA + 10 wt. % X-V-2 | 6.91 | 0.76 | 6.35 | 0.78 | 6.43 | 0.76 |
| 624D | NaVSA + 20 wt. % X-V-2 | 6.50 | 0.78 | 6.20 | 0.84 | 5.95 | 0.81 |
| 628A | FAA + 2.5 wt.% X-V-1 | 10.44 | 0.96 | 9.76 | 0.98 | 2.92 | 0.50 |
| 628A | FAA + 2.5 wt.% X-V-1 | | | 9.85 | 0.97 | 3.45 | 0.50 |
| 628B | FAA + 5.0 wt.% X-V-1 | 10.22 | 1.01 | 9.61 | 1.01 | 2.93 | 0.48 |
| 628C | FAA + 10 wt.% X-V-1 | 10.05 | 1.02 | 9.36 | 1.02 | 2.84 | 0.47 |
| 628C | FAA + 10 wt.% X-V-1 | 10.68 | 0.98 | 9.18 | 0.97 | 2.85 | 0.42 |
| 628C | FAA + 10 wt.% X-V-1 | 9.87 | 0.93 | 9.63 | 0.85 | 2.13 | 0.27 |
| 628D | FAA + 20 wt.% X-V-1 | 9.12 | 1.03 | 8.52 | 1.02 | 2.59 | 0.50 |
| 629A | FAA + 25mol% NaOH + 12.5 wt. % X-V-1 | 9.59 | 1.02 | 9.18 | 1.00 | 2.87 | 0.44 |
| | | 10.27 | 0.99 | 9.52 | 0.98 | 2.79 | 0.41 |
| 629B | FAA + 50mol% NaOH + 12.5 wt. % X-V-1 | 9.58 | 1.02 | 9.05 | 1.02 | 2.69 | 0.38 |
| 629B | FAA + 50mol% NaOH + 12.5 wt. % X-V-1 | 10.06 | 0.93 | 9.01 | 0.85 | 1.68 | 0.14 |
| 629C | FAA + 75mol% NaOH + 12.5 wt. % X-V-1 | 9.41 | 0.98 | 9.33 | 1.01 | 3.19 | 0.54 |
| 629D | FAA + 100mol% NaOH + 12.5 wt. % X-V-1 | 9.55 | 0.98 | 9.43 | 1.00 | 3.05 | 0.54 |
| 636A2 | NaVSA + 5 wt.% X-V-3 | | | 6.43 | 0.72 | 7.15 | 0.75 |
| 636A3 | NaVSA + 10 wt. % X-V-3 | 7.93 | 0.77 | 6.70 | 0.76 | 7.07 | 0.77 |
| 636A4 | NaVSA + 20 wt. % X-V-3 | 7.41 | 0.76 | 6.29 | 0.76 | 6.28 | 0.75 |
| 636B3 | NaVSA + 10 wt. % X-V-3 | 9.52 | 0.81 | 6.49 | 0.74 | 7.03 | 0.77 |
| 636B4 | NaVSA + 20 wt. % X-V-3 | 7.76 | 0.79 | 6.10 | 0.77 | 6.53 | 0.78 |
| 639A | FAA + 10 wt.% X-V-1 | 9.72 | 0.92 | 8.75 | 0.84 | 3.20 | 0.41 |
| 639A | FAA + 10 wt.% X-V-1 | 10.38 | 0.90 | 9.45 | 0.85 | 1.92 | 0.22 |

(continued)

| Sample Name | Description | Total mmoles ($Na^+$ +$K^+$) bound/gm resin, pH12.5 | $Na^+$:$K^+$ ratio at pH12.5 | Total mmoles ($Na^+$ + $K^+$) bound/gm resin, pH 6.25 | $Na^+$ :$K^+$ ratio at pH 6.25 | Total mmoles ($Na^+$ + $K^+$) bound/gm resin. pH3 | $Na^+$: $K^+$ratio at pH3 |
|---|---|---|---|---|---|---|---|
| 639B | FAA + 50mol% NaOH + 12.5 wt. % X-V-1 | 8.97 | 0.92 | 8.85 | 0.85 | | |
| 639B | FAA + 50mol% NaOH + 12.5 wt. % X-V-1 | 9.46 | 0.95 | 8.68 | 0.83 | 1.73 | 0.17 |
| 639B | FAA + 50mol% NaOH + 12.5 wt. % X-V-1 | 8.447 | 0.87 | 8.192 | 0.834 | | |
| 616B3 | NaVSA + 20 wt. % X-V-1 | 5.87 | 0.71 | 6.14 | 0.72 | 6.57 | 0.78 |
| 100851A2 | purified NaVSA + 5 wt.% X-V-1 | 5.92 | 0.67 | 6.68 | 0.70 | 5.58 | 0.69 |
| 100851A2 | purified NaVSA + 5 wt.% X-V-1 | 7.42 | 0.79 | 7.08 | 0.74 | 5.99 | |
| 100851A2 | purified NaVSA + 5 wt.% X-V-1 | 6.57 | 0.77 | 6.45 | 0.71 | 5.87 | 0.74 |
| 100851A3 | purified NaVSA + 10 wt.% X-V-1 | 6.27 | 0.07 | 6.84 | 0.72 | 6.17 | 0.72 |
| 100851A3 | purified NaVSA + 10 wt.% X-V-1 | 6.97 | 0.75 | 7.50 | 0.74 | 6.78 | 0.77 |
| 100851A4 | purified NaVSA + 20 wt.% X-V-1 | 5.84 | 0.71 | 6.53 | 0.73 | 5.21 | 0.70 |
| 100851A4 | purified NaVSA + 20 wt.% X-V-1 | 6.28 | 0.81 | 6.28 | 0.75 | | |
| 100851A4 | purified NaVSA + 20 wt.% X-V-1 | 6.22 | 0.76 | 6.82 | 0.75 | 5.48 | 0.74 |
| 100851B1 | purified NaVSA + 2.5 wt.% X-V-5 | 6.42 | 0.65 | 6.50 | 0.65 | 6.09 | 0.65 |
| 100851B2 | purified NaVSA + 5 wt.% X-V-5 | 5.76 | 0.62 | 6.72 | 0.64 | 6.27 | 0.65 |
| 100851B2 | purified NaVSA + 5 wt.% X-V-5 | 6.77 | 0.73 | 7.27 | 0.67 | 6.48 | 0.71 |
| 100851B3 | purified NaVSA + 10 wt.% X-V-5 | 5.83 | 0.61 | 7.07 | 0.64 | 5.57 | 0.60 |
| 100851B3 | purified NaVSA + 10 wt.% X-V-5 | 6.66 | 0.80 | 7.27 | 0.69 | 6.05 | 0.68 |
| 100851B4 | purified NaVSA + 20 wt.% X-V-5 | 6.50 | 0.65 | 6.25 | 0.61 | 5.22 | 0.59 |
| 100851B4 | purified NaVSA + 20 wt.% X-V-5 | 5.50 | 0.66 | 6.59 | 0.66 | 5.82 | 0.66 |
| 100851C2 | purified NaVSA + 5 wt.% X-V-1 | 6.52 | 0.70 | 6.40 | 0.68 | 5.52 | 0.67 |
| 100851C2 | purified NaVSA + 5 wt.% X-V-1 | 7.23 | 0.78 | 7.03 | 0.75 | | |
| 100851C3 | purified NaVSA + 10 wt.% X-V-1 | 6.77 | 0.72 | 7.02 | 0.72 | 5.90 | 0.71 |

(continued)

| Sample Name | Description | Total mmoles ($Na^+$ +$K^+$) bound/gm resin, pH12.5 | $Na^+$:$K^+$ ratio at pH12.5 | Total mmoles ($Na^+$ + $K^+$) bound/gm resin, pH 6.25 | $Na^+$ :$K^+$ ratio at pH 6.25 | Total mmoles ($Na^+$ + $K^+$) bound/gm resin. pH3 | $Na^+$: $K^+$ratio at pH3 |
|---|---|---|---|---|---|---|---|
| 100851C4 | purified NaVSA + 20 wt.% X-V-1 | 6.05 | 0.72 | 6.08 | 0.71 | 4.66 | 0.68 |
| 100851C4 | purified NaVSA + 20 wt.% X-V-1 | 6.51 | 0.78 | 8.07 | 0.80 | | |
| 100851D1 | purified NaVSA + 2.5 wt.% X-V-5 | 7.07 | 0.74 | 7.28 | 0.71 | 5.87 | 0.69 |
| 100851D1 | purified NaVSA + 2.5 wt.% X-V-5 | 7.65 | 0.73 | 7.40 | 0.72 | | |
| 100851D2 | purified NaVSA + 5 wt.% X-V-5 | 6.83 | 0.66 | 7.17 | 0.71 | 5.42 | 0.64 |
| 100851D2 | purified NaVSA + 5 wt.% X-V-5 | 7.91 | 0.75 | 7.37 | 0.70 | | |
| 100851D3 | purified NaVSA + 10 wt.% X-V-5 | 6.70 | 0.67 | 6.87 | 0.66 | 5.21 | 0.64 |
| 100851D4 | purified NaVSA + 20 wt.% X-V-5 | 6.24 | 0.67 | 6.46 | 0.67 | 6.63 | 0.58 |
| 100851D4 | purified NaVSA + 20 wt.% X-V-5 | 7.01 | 0.68 | 6.61 | 0.70 | | |
| 100982A1 | FAA + 10 wt.% X-V-1 | 9.66 | 0.89 | 9.02 | 0.86 | 3.40 | 0.50 |
| 100982A1 | FAA + 10 wt.% X-V-1 | | | 8.47 | 0.86 | | |
| 100982A2 | 90 wt.% FAA + 10 wt.% acrylic acid + 10 wt.% X-V-1 | 9.81 | 0.92 | 8.49 | 0.86 | 2.98 | 0.52 |
| 100982A2 | 90 wt.% FAA + 10 wt.% acrylic acid + 10 wt.% X-V-1 | | | 8.00 | 0.86 | | |
| 100982A3 | 80 wt.% FAA + 20 wt.% acrylic acid + 10 wt.% X-V-1 | 10.00 | 0.95 | 7.97 | 0.86 | 2.89 | 0.56 |
| 100982A3 | 80 wt.% FAA + 20 wt.% acrylic acid + 10 wt.% X-V-1 | | | 7.74 | 0.87 | | |
| 100982A4 | 70 wt.% FAA + 30 wt.% acrylic acid + 10 wt.% X-V-1 | 9.92 | 0.97 | 8.52 | 0.85 | 2.42 | 0.54 |
| 100982A4 | 70 wt.% FAA + 30 wt.% acrylic acid + 10 wt.% X-V-1 | | | 7.49 | 0.88 | | |

(continued)

| Sample Name | Description | Total mmoles ($Na^+$ +$K^+$) bound/gm resin, pH12.5 | $Na^+$:$K^+$ ratio at pH12.5 | Total mmoles ($Na^+$ + $K^+$) bound/gm resin, pH 6.25 | $Na^+$ :$K^+$ ratio at pH 6.25 | Total mmoles ($Na^+$ + $K^+$) bound/gm resin. pH3 | $Na^+$: $K^+$ratio at pH3 |
|---|---|---|---|---|---|---|---|
| 100982A5 | 60 wt.% FAA + 40 wt.% acrylic acid + 10 wt.% X-V-1 | 10.00 | 1.00 | 7.48 | 0.86 | 2.01 | 0.53 |
| 100982A5 | 60 wt.% FAA + 40 wt.% acrylic acid + 10 wt.% X-V-1 | | | 7.10 | 0.89 | | |
| 100982A6 | 50 wt.% FAA + 50 wt.% acrylic acid + 10 wt.% X-V-1 | 10.41 | 1.03 | 7.56 | 0.87 | 2.11 | 0.61 |
| 100982A6 | 50 wt.% FAA + 50 wt.% acrylic acid + 10 wt.% X-V-1 | | | 7.11 | 0.90 | | |
| 101012A1 | purified NaVSA + 2.5 wt.% X-V-2 | | | | | | |
| 101012A2 | purified NaVSA + 5 wt.% X-V-2 | 7.50 | 0.74 | 7.70 | 0.74 | 6.49 | 0.74 |
| 101012A3 | purified NaVSA + 10 wt.% X-V-2 | 7.04 | 0.74 | 7.31 | 0.74 | 6.27 | 0.74 |
| 101012A4 | purified NaVSA + 20 wt.% X-V-2 | 6.52 | 0.75 | 6.88 | 0.75 | 6.01 | 0.76 |
| 101012B1 | purified NaVSA + 2.5 wt.% X-V-4 | | | | | | |
| 101012B2 | purified NaVSA + 5 wt.% X-V-4 | 7.53 | 0.71 | 7.64 | 0.71 | 6.93 | 0.72 |
| 101012B3 | purified NaVSA + 10 wt.% X-V-4 | 6.88 | 0.70 | 7.19 | 0.71 | 6.24 | 0.70 |
| 101012B4 | purified NaVSA + 20 wt.% X-V-4 | 6.34 | 0.68 | 6.78 | 0.70 | 6.08 | 0.70 |
| 101012D1 | purified NaVSA + 2.5 wt.% X-V-7 | 7.02 | 0.73 | 6.68 | 0.73 | 4.86 | 0.67 |
| 101012D2 | purified NaVSA + 5 wt.% X-V-7 | 7.35 | 0.74 | 7.24 | 0.74 | 6.58 | 0.73 |
| 101012D3 | purified NaVSA + 10 wt.% X-V-7 | 7.17 | 0.74 | 7.30 | 0.74 | 6.64 | 0.75 |
| 101012D4 | purified NaVSA + 20 wt.% X-V-7 | 6.33 | 0.72 | 6.64 | 0.74 | 5.83 | 0.74 |
| 101028A1 | purified NaVSA + 10 wt.% X-V-1 | 6.47 | 0.76 | 5.69 | 0.75 | 5.47 | 0.77 |

(continued)

| Sample Name | Description | Total mmoles ($Na^+$ +$K^+$) bound/gm resin, pH12.5 | $Na^+$:$K^+$ ratio at pH12.5 | Total mmoles ($Na^+$ + $K^+$) bound/gm resin, pH 6.25 | $Na^+$ :$K^+$ ratio at pH 6.25 | Total mmoles ($Na^+$ + $K^+$) bound/gm resin. pH3 | $Na^+$: $K^+$ratio at pH3 |
|---|---|---|---|---|---|---|---|
| 101028A2 | 90 wt.% purified NaVSA + 10 wt. % FAA +10 wt. % X-V-1 | 6.67 | 0.81 | 6.01 | 0.79 | 4.67 | 0.72 |
| 101028A3 | 80 wt.% purified NaVSA + 20 wt. % FAA +10 wt. % X-V-1 | 7.17 | 0.82 | 6.50 | 0.80 | 4.25 | 0.68 |
| 101028A4 | 70 wt.% purified NaVSA + 30 wt. % FAA +10 wt. % X-V-1 | 7.33 | 0.84 | 6.77 | 0.81 | 4.12 | 0.66 |
| 101028A5 | 60 wt.% purified NaVSA + 40 wt. % FAA +10 wt. % X-V-1 | 7.69 | 0.85 | 7.00 | 0.83 | 3.43 | 0.60 |
| 101028A6 | 50 wt.% purified NaVSA + 50 wt. % FAA +10 wt. % X-V-1 | 8.25 | 0.87 | 7.29 | 0.85 | 3.80 | 0.63 |
| 101029A2 | VPA + 5 wt.% X-V-1 | | | | | | |
| 101029A3 | VPA + 10 wt.% X-V-1 | 11.38 | 1.49 | 5.70 | 1.00 | 2.37 | 0.89 |
| 101029A4 | VPA + 20 wt.% X-V-1 | 10.15 | 1.66 | 4.90 | 1.03 | 2.27 | 0.88 |
| 101029B2 | VPA + 50mol% NaOH + 5 wt.% X-V-1 | | | | | | |
| 101029B3 | VPA + 50 mol% NaOH + 10 wt. % X-V-1 | 10.97 | 1.50 | 5.27 | 0.98 | 2.63 | 0.91 |
| 101029B4 | VPA + 50mol% NaOH + 20 wt. % X-V-1 | 10.23 | 1.62 | 5.10 | 1.01 | 2.06 | 0.88 |
| 684A | FAA + 5 wt.% X-V-1 | 10.7 | 0.91 | 10.30 | 0.84 | nm | nm |
| 684B | FAA + 5 wt.% X-V-1 | 9.80 | 0.83 | 9.70 | 0.82 | nm | nm |
| | Dowex 50WX4-200 (average of 15 experiments) | 5.37 | 0.77 | 5.51 | 0.77 | 4.92 | 0.76 |
| | Dowex 50W (Standard deviation of 15 experiments) | 0.77 | 0.06 | 0.81 | 0.08 | 0.80 | 0.06 |

nm: not measured

These examples show that the polymers of the invention display high potassium binding capacity at physiological pHs. In particular polymers prepared from 2-fluoroacrylic acid can bind up to two times more potassium than sulfonated polystyrene resins Dowex.

***Titration curves of alpha-fluoroacrylate copolymer with acrylic acid from Table 11***

The protocol was as per Helfferich, F. "Ion Exchange" (1962) McGraw-Hill, New York).

1. Approximately 50mg of polymer (acid-form) was measured into 15x100mm glass test tubes.
2. The volume of 1 M NaOH required to generate the required mEq was calculated, and enough water was added to the tubes to keep the ratio of solution volume to resin weight constant.
3. The required mEq of NaOH was added to the polymer from a 1 M NaOH stock.
4. The tubes were sealed and rotated for 4 days to allow to come to equilibrium
5. The equilibrated pH was measured while continuing to mix.

The results are shown in Figure 16. This example shows that polyalpha-fluoroacrylate has a lower pKa (equal to pH value at half-neutralization) than a methacrylic containing ion-exchange resin such as Amberlite CG50. The pKa value for the FAA gel material (100982A1 from Table 11) can be estimated from Figure 18 at about 5.6 versus 8 for Amberlite CG50. The incorporation of acrylic acid tends to increase pKa in proportion to the wt-% of acrylic acid in the FAA-Acrylic acid copolymer. This indicates that an electro-withdrawing group such as fluorine in the alpha position to COOH decreases the pKa and increases the overall binding capacity within the typical physiological pH range of 5-7.

**Example 3: Procedure for predicting binding of cations in the human GI**

This procedure was used to model the conditions of use of a potassium binder drug and measure the binding characteristics of the polymer for potassium (target solute) in the presence of other competing cations. A meal mimic was prepared and artificially digested in the presence of pepsin and pancreatic juice. The sequence of addition of enzymes and the pH profile were controlled so that the digestion process was simulated down to the jejunum level. The test polymers, preloaded with lithium, were added to the digested meal mimic and allowed to equilibrate for a fixed period of time; the mixture was then centrifuged and the supernatant was assayed for $Na^+$, $K^+$, $NH_4^+$, $Ca^{2+}$, and $Mg^{2+}$ by ion chromatography. The lithium released was computed as the total cation exchange, while the decrease in concentrations of the other cations was used to compute their binding variations in western diets.

***Preparation of Resin***

Resin (test resin, or Dowex 50WX4-200 used as a comparative), was washed extensively in 1 M HCl to convert it to the H-form. It was then washed extensively in 1 M LiOH. Excess LiOH was removed by washing in $ddH_2O$. The resins were lyophilized and stored in a desiccator.

Figure 1 depicts starting cation concentrations in meal mimic and Figure 2 depicts binding of cations by resins in meal mimic.

***Measurement of binding capacities in cecal and fecal extracts***

Two volumes (w/v) of ice-cold $ddH_2O$ were added to the human feces and to normal rabbit cecal contents. These were incubated with rotation at 4°C with end-over-end rotation for at least 1hour to extract soluble cations. Fecal and cecal extracts, as well as thawed meal mimics, were centrifuged at 2000g for 10 minutes to clarify. Approximately 50mg Li-form Dowex 50W was weighed into 16x100mm glass test tubes. Control test tubes were included that contained no resin. Clarified extracts or mimics were added to a final resin concentration of 2.5mg/ml. 5-10ml of extracts or mimic were added to the control test tubes. Tubes were sealed and rotated at 4°C for 90 minutes. The tubes were centrifuged at 500g for thirty minutes to precipitate the resin. Supernatant samples were taken. The samples were then prepared for ion chromatography by spinning at 13,000g for ten minutes, taking the supernatant and rapidly passing across a 3000Da cutoff dialysis membrane by centrifugation. Extracts were further diluted 1:5 (v/v) in $ddH_2O$ before applying to the IC columns. Start (without resin) and equilibrium (with resin) concentrations of $Li^+$, $Na^+$, $K^+$, $NH_4^+$, $Ca^{++}$ and $Mg^{++}$ were determined, and the amount (in mmoles cation/gm resin) of $Li^+$ released, as well as $Na^+$, $K^+$, $NH_4^+$, $Ca^{++}$ and $Mg^{++}$ bound were calculated.

***Procedure for measuring the binding* of *cations by resins in human fecal extracts***

Resins and feces were prepared as follows. Resins were washed extensively in 1 M HCl to convert them to the H-form. Excess HCl was removed by washing in $ddH_2O$. The resins were lyophilized and stored in a desiccator. Fecal samples were obtained from two human subjects, frozen immediately and stored at -80°C to minimize ammonium production *ex vivo.*

All experiments were performed in triplicate. Error bars on Figures 3 and 4 indicate standard deviations values. Fecal samples were resuspended in two volumes of ice-cold $ddH_2O$ (w/v) and incubated overnight at 4°C to extract soluble cations. The extract was then clarified by centrifuging at 2000g for ten minutes. H-form resins were weighed into disposable 15ml-capacity columns. They were them washed extensively in 150mM LiOH to convert them to the Li-form. They were washed in $ddH_2O$ to remove excess LiOH. Clarified fecal extract was applied to the columns to a final resin concentration of 2.5mg/ml of extract. A sample was retained for calculating resin concentrations in the absence of resin. Columns were capped and rotated at 44°C for three hours. They were then eluted by centrifugation into 50ml polypropylene tubes. The pH of eluted extracts and retained clarified fecal extracts were measured (it had not changed: Sample 1 pH was 6.75, sample 2 pH was 7.1). The samples were then prepared for ion chromatography by spinning at 13,000g for ten minutes, taking the supernatant and rapidly passing across a 3000Da cutoff dialysis membrane by centrifugation. Extracts were further diluted 1:5 (v/v) in $ddH_2O$ before applying to the IC columns. Start (without resin) and equilibrium (with resin) concentrations of $Li^+$, $Na^+$, $K^+$, $NH_4^+$, $Ca^{++}$ and $Mg^{++}$ were determined, and the amount (in mmoles cation/gm resin) of $Li^+$ released, as well as $Na^+$, $K^+$, $NH_4^+$, $Ca^{++}$ and $Mg^{++}$ bound were calculated. In Figure 4 "Total occupied" refers to the sum of $Li^+$ (i.e. monovalent) binding sites occupied by the other cations, taking into account the divalent nature of $Ca^{++}$ and $Mg^{++}$.

Data presented in Figure 4 demonstrate that the ex-vivo binding of potassium in human fecal extracts for the FAA based material is about twice as much that of Dowex 50WX4-200 (a material essentially identical in composition to the potassium binder Kayexalate). The ex-vivo binding of potassium by the Dowex resin is essentially the same as that reported for polystyrene sulfonate resins in human clinical studies, which establishes this method as a good predictor for in-vivo binding performance. It also indicates that other cations, in particular Magnesium and Calcium, compete with potassium for the binding sites of the polymers. Figure 3 depicts the original concentrations of cations in the Feces of Subject 1 and Subject 2. Figure 4 depicts the binding of cations in human fecal extracts to cation exchange resins.

**Example 4: Method of selection of semi-permeable membrane with high potassium binding selectivity over magnesium and calcium**

This protocol describes a method to optimize polymeric materials with regards to their ion permselectivity characteristics, which then can be used as the shell component for the making of potassium selective core-shell ion-exchange particles.

***Polymer synthesis and membrane preparation:***

Polymeric membrane materials with different compositions were prepared by radical copolymerization of DBA (N, N'-dibutyl acrylamide) and DEAEMA (N,N'-diethylaminoethylmethacrylate) in a glove box using miniaturized reactors in a library format. AIBN was used as the initiator and ethanol as the solvent. Polymers were isolated by precipitation into water, freeze-dried, and characterized by GPC and H-NMR. The composition of the polymer (DBA mol%) ranges from 30% to 70% and molecular weight ranges from 200K to 300K as shown below:

Table 14

| **Polymer ID 101224** | D1 | D2 | D3 | D4 | D5 | D6 |
|---|---|---|---|---|---|---|
| Mn ($x10^3$) | 327 | 326 | 322 | 285 | 240 | 217 |
| Mw ($x10^3$) | 584 | 563 | 520 | 467 | 411 | 340 |
| PDI | 1.78 | 1.73 | 1.61 | 1.64 | 1.71 | 1.56 |
| Composition (DBA, mol%) | 31.2 | 37.1 | 48.5 | 56.1 | 64.4 | 68.5 |

Polymer membranes were prepared by casting a 2-wt% toluene solution of DBA-co-DEAEMA onto a regenerated cellulose dialysis membrane (RC membrane with MWCO of 14 K). After toluene was evaporated, a polymer membrane was formed on the top of dialysis membrane. A composite membrane of polymer membrane and RC membrane

was thus prepared.

***Permeability study on cations***

The composite membrane was first clamped onto a glass tube with diameter of 13 mm, and then immersed into a 2 L of donor solution of cations. The tube was filled with 10 ml of acceptor solution (lactose solution with the same osmolality as the donor solution (240mM)). The acceptor solution was sampled at a specified time interval and analyzed by ion chromatography. See Figure 5.

Donor solution was prepared by mixing the aqueous solution of NaCl, KCl, $CaCl_2.2H_2O$, and $MgSO_4.7H_2O$. The solution was buffered to pH 6 by using 14 mM of MES (2-[N-morpholine]ethanesulfonic acid] solution. The concentrations of different cations determined by IC were as follows: [$Na^+$], 40.46 mM; [$K^+$], 31.44 mM; [$Mg^{2+}$], 33.25 mM; [$Ca^{2+}$], 22.324 mM.

Determination of the permeability coefficient (P) of different cations: As mentioned in the measurement set-up, the acceptor solution was sampled at a specific time interval and analyzed by IC. Assuming a Fick's first law of diffusion, P is readily obtained by linearization of the data, following a method of calculation reported in equation 1 in G. Van den Mooter, C. Samyn, and R. Kinget, International Journal of Pharmaceutics, 111, 127-136(1994). The permeability coefficients of different cations were thus calculated from the slope from this linear relationship.

$$-\ln\left(\frac{C_o - C_a}{C_o}\right) = \frac{PS}{Va}t \qquad \text{...........Equation 1}$$

Where $C_o$ is the initial concentration of the solute in the donor compartment and $C_a$ the concentration in the acceptor compartment at time t, Va is the volume of the acceptor compartment, and S the surface of the membrane.

Permselectivity: As described above, the permeability coefficient was calculated for each cation. By normalizing the permeability coefficient of $Na^+$ as 1, the permselectivity for cations M1 and M2 can be calculated as follows: $P_{M1}^{M2} = P(M2)/P(M1)$

***Permeability coefficients of different cations through different membranes:***

Table 14 shows the permeability coefficients of different cations at different membranes. When polymers are more hydrophilic (Polymer D3 and D4 with DBA% 48.5 and 56.1%, respectively), all cations, such as $Na^+$, $K^+$, $Mg^{2+}$, and $Ca^{2+}$, are more permeable and their permeability coefficients are comparable to those through a blank dialysis membrane (RC membrane) and reflect the self-diffusivity of the cations. However, with the increasing DBA content in polymer membrane (See Table 15 for D5 and D6), the permeability coefficients of different cations decreased as compared with blank membrane, which means that the hydrophobic nature of polymer membrane could make cations less permeable through the hydrophobic barrier.

Table 15: Permeability coefficients of cations at different membranes

| Polymer ID | DBA (mol%) | $PNa^+$ (cm/sec) | $PK^+$ (cm/sec) | $PMg^{2+}$ (cm/sec) | $PCa^{2+}$ (cm/sec) |
|---|---|---|---|---|---|
| D3 | 48.5 | 2.41(±0.26)E-4 | 3.11(±0.34)E-4 | 6.50(±0.08)E-5 | 6.0(±0.07)E-5 |
| D4 | 56.1 | 4.28(±0.44)E-5 | 6.11(±0.61)E-4 | 1.13(±0.11)E-5 | 1.04(±0.05)E-5 |
| D5 | 64.4 | 4.32(±0.20)E-6 | 5.79(±3.59)E-6 | 5.42(±4.11)E-7 | 3.32(±3.33)E-7 |
| D6 | 68.5 | 1.50(±0.05)E-7 | - | - | - |

Another characteristic for the permeability of different cations is their permselectivity. By normalizing the value of $P_{Na+}$ as 1, the permselectivity for other cations can be calculated and the results are shown in Table 16. The permselectivity of $P_{Mg}/P_{Na}$ and $P_{Ca}/P_{Na}$ decreases with the increasing DBA content in polymer membranes, which implies that more hydrophobic polymer membranes may have better selectivity for different cations. For a better selectivity for different cations, two factors should be considered - the charge density and the membrane hydrophobicity.

TABLE 16

| Polymer ID | DBA(%) | P(K+)/P(Na+) | P(Ca2+)/P(Na+) | P(Mg2+)/P(Na+) | P(K+)/P(Mg2+) |
|---|---|---|---|---|---|
| D3 | 48.5 | 1.29 | 0.27 | 0.25 | 5.16 |
| D4 | 56.1 | 1.43 | 0.26 | 0.24 | 5.96 |
| D5 | 64.4 | 1.34 | 0.13 | 0.08 | 16.75 |

**Example 5: Synthesis of poly-2-fluoroacrylic acid beads**

Beads are prepared by a direct suspension process where a mixture of 2-fluoroacrylic methyl ester/divinylbenzene/benzoyl peroxide in a weight ratio 90/9/1 are dispersed in water under high shear with polyvinylalcohol as a suspending agent. The suspension is stirred and heated at 80°C for 10hours. The residual monomer is eliminated by steam stripping. The beads are then filtered and treated with aqueous 3M NaOH to hydrolyze the polymer, then washed, treated with HCL, water-washed, and finally dried to form the desired polyα-fluoroacrylic acid particles. The average bead diameter is 250 microns as measured by Master Sizer (Malvern UK).

**Example 6: Preparation of poly-2-fluornacrylic acid / core- (DBA-DEAEMA) / shell particles**

The core-shell particles are prepared by forming a coating of polymer D2 on the poly-2-fluoroacrylic acid beads prepared in example 5 using a Wurster coater. The shell polymer prepared in example 4 is first dissolved at 20 wt-% in toluene, and the thus obtained solution then dispersed in water in a 1: 4 weight ratio with 2 wt-% based on the organic phase of CTAB (HexadecyltrimethylAmmonium Bromide) as a surfactant, using a Ultra-Turrax high-shear homogeneizer. The toluene is then driven off by evaporation under reduced pressure. The average diameter of the dispersion particles is 0.3 micrometer, as measured by Dynamic Light Scattering. The poly-2-fluoroacrylic acid beads are spray-coated with the shell polymer dispersion using a Wurster fluid bed coater 2"- 4"/6" Portable Unit. The fluidized bed unit is operated so that an average 5 microns thick coating is deposited on the core particles.

The potassium binding capacity when measured in a fecal extract as described in Example 3 is expected to be twice higher than that measured with the uncoated poly-α-fluoroacrylic acid beads.

**Reference Example 7: Preparation of polystyrene sulfonate/core- polyethyleneimine shell particles with Na+ and K+ selective-binding propertie**

*Procedure for coating PEI on Dowex beads*

PEI (poly(ethyleneimine), Mw10,000) and Dowex beads (H-form, X4-200) were purchased from commercial sources. PEI aqueous solutions with different concentrations were prepared by dissolving PEI directly into nanopure water.

Weighed dried Dowex beads were mixed with PEI aqueous solution in library format glass tubes. After a specified reaction time, the tubes were sealed and centrifuged at 1000 rpm for 15 minutes, the supernatant solutions were then decanted off. To the beads in each tube was added nanopure water to a total volume of 10 ml and all tubes were sealed and tumbled for 30 minutes. The same tumbling-centrifuging was repeated 3 times. The beads were freeze-dried and weighted until a constant weight was obtained.

The reaction solution composition and gel weight increase are displayed in Table 17.

**Table 17: Conditions for coating PEI on Dowex beads**

| Dowex Bead Weight (gm) | PEI Conc. (wt%) | PEI volume (ml) | Reaction time (hours) | Coated bead ID | Weight increase (Δwt%) |
|---|---|---|---|---|---|
| 0.1274 | 2.5 | 10 | 1 | DOWEX(2.5wt-1h) | * |
| 0.2223 | 2.5 | 10 | 6 | DOWEX(2.5wt-6h) | 3.1 |
| 0.1609 | 1.5 | 10 | 1 | DOWEX(2.5wt-1h) | * |
| 0.2407 | 1.5 | 10 | 6 | DOWEX(2.5wt-6h) | 0.9 |

(continued)

| Dowex Bead Weight (gm) | PEI Conc. (wt%) | PEI volume (ml) | Reaction time (hours) | Coated bead ID | Weight increase (Δwt%) |
|---|---|---|---|---|---|
| 0.2016 | 0.5 | 10 | 1 | DOWEX(2.5wt-1h) | * |
| 0.2347 | 0.5 | 10 | 6 | DOWEX(2.5wt-6h) | * |
| * No weight increase was observed. | | | | | |

*Method for binding study*

A mixture of NaCl, KCl, $MgCl_2$, and $CaCl_2$ was dissolved in a MES buffer (pH6.0) (MES, 2-[N-morpholine] ethanesulfonic acid]. The concentration for each cation was determined by IC. The concentrations for $Na^+$, $K^+$, $Mg^{2+}$, and $Ca^{2+}$ are 26.4 mM, 9.75 mM, 4.75 mM and 4.16 mM respectively.

Weighed dried PEI-coated bead was put into a tube which contains 5-ml of MES buffer solution of NaCl, KCl, $MgCl_2$, and $CaCl_2$. The tube was sealed and tumbled. After a certain period of time as indicated in figure 6, the tube was centrifuged. 100 microliter of solution was then taken out from the supernatant for IC analysis. The binding amount of PEI coated beads for different cations were calculated from the concentration change in the solution.
The calculation is as follows:

$$\text{Ion bound in beads (mmol/g)} = [V \times (C_0 - C_t) /\{[\text{weight of beads}] \times 1000\}$$

$C_0$: initial concentration of metal ion (in mM)
$C_t$: concentration of metal ion after bead binding at a certain time (t hrs) (in mM)
V: solution volume (5 ml)
Weight of beads (gm)

The binding data of different PEI coated beads for different cations are shown in Figure 6. PEI coated Dowex beads show higher $Na^+$ and $K^+$ binding than the uncoated beads (bare beads). The coated beads show much more selective binding than bare beads. The thicker the PEI coating (e.g. Dowex (2.5wt-6h), coated from 2.5 wt% PEI solution for 6 hours), the more selective for the different cations. The binding kinetic study shows that the binding of cations equilibrates faster for the thinner coated beads and bare beads.

**Reference Example 8: Polystyrene sulfonate beads with Eudragit shell**

Shell material: Eudragit RL100 (Rohm), a copolymer of acrylic and methacrylic acid esters with 8.85-11.96% cationic ammonio methacrylate units, 10 wt% in ethanol and 10wt% triacetin. Core: Lewatit (cross-linked polystyrene sulfonate in sodium form), size - 300 μm.

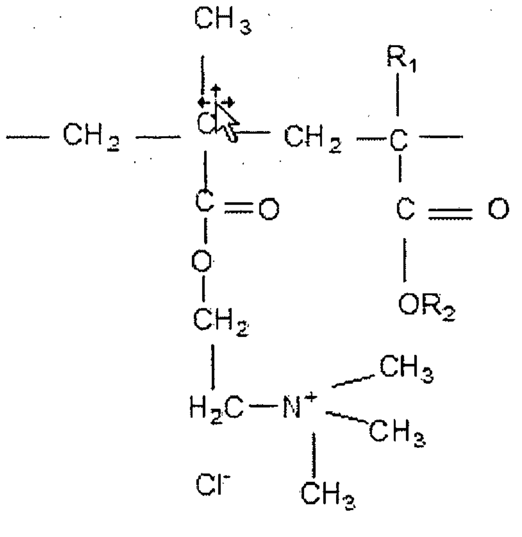

RL 100

$R_1 = H, CH_3$
$R_2 = CH_3, C_2H_5$

The shell was applied using a FluidAir Wurster coater.

Binding was measured under following conditions:

Donor solution: 50 mM KCl and 50 mM $MgCl_2$
Bead concentration: 4 mg/ml
Duration: 6 hours

Figure 7 shows the effect of the shell on $Mg^{2+}$ and $K^+$ binding. With increasing ratio of shell to core, $Mg^{2+}$ binding decreased and $K^+$ binding increased. 20 wt% shell coating gave a $K^+$ binding capacity of 1.65 meq/gm, which is about 3 times higher than for uncoated Dowex.

**<u>Reference Example 9: Polystyrene sulfonate beads with benzylated polyethylene imine shell</u>**

***<u>Synthesis</u>* <u>of</u> *<u>benzylated polyethyleneimine</u>* <u>(</u>*<u>PEI</u>*<u>)</u>**

To a 250 ml of round bottom flask were charged 15.8 g of PEI (363 mmol of $-NH_2$) and 126 ml of ethanol, this mixture was magnetically stirred until PEI was completely dissolved, then 30 g of $NaHCO_3$ (FW, 84; 256 mmol) and 40 ml of benzyl chloride (363 mmol) were subsequently added. The above mixture was reacted at 55°C under nitrogen atmosphere overnight. Dichloromomethane was added to the slurry reaction mixture, followed by filtration to remove inorganic salt. The solvent in filtrate was removed by vacuum. Dicholromethane was used again to re-dissolve the reaction product; inorganic salt was further removed by filtration. The solvent in the filtrate was removed again under vacuum. Finally, the product was triturated in hexane, filtered and washed with hexane, and dried under vacuum. The benzylation degree was 84% as determined by $^1$HNMR. Similar materials with various degree of benzylation (respectively 20% and 40% for Ben(20) and Ben(40)) were prepared by adjusting the benzyl chloride to PEI ratio.

Benzylated polyethylene imine (Ben-PEI) was coated onto Dowex beads.

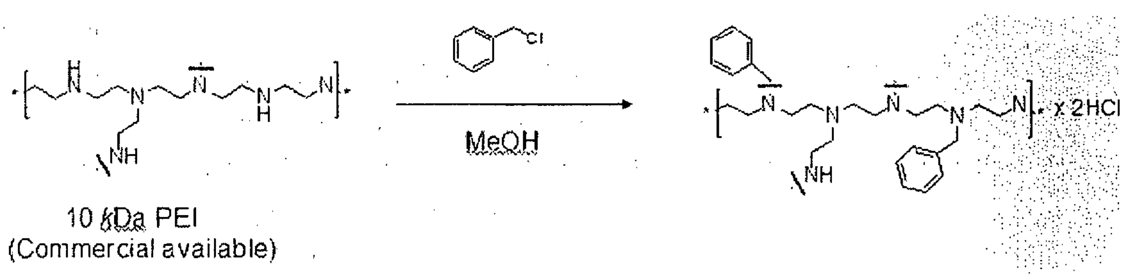


The shell was coated using solvent coacervation. The shell Ben(84)-PEI was dissolved in methanol and water mixture (3:1) at pH of 3. Shell and core were mixed for 5 minutes and methanol was removed by rotovap (40 minutes), isolated, washed, and dried.

Binding was measured under following conditions:

Donor solutions: 50 mM KCl and 50 mM $MgCl_2$
Bead concentration: 4 mg/ml
Duration: 6 and 24 hours

Results of the binding measurements are shown in Figure 8. Ben(84)-PEI showed selective binding for potassium after 6 and 24 hours as revealed by lower $Mg^{2+}$ binding compared to naked beads.

Figure 9 depicts the stability of Ben(84)-PEI coated Dowex (K) beads under acid conditions representative of the acidic conditions in the stomach. The beads were exposed to pH 2 HCl for 6 hours, isolated, and dried. Binding selectivity was tested for the post-treated beads. Binding conditions were as follows:

Donor solutions: 50 mM KCl and 50 mM $MgCl_2$
Bead concentration: 4 mg/ml
Duration: 6 and 24 hours
The coating was stable and binding selectivity was maintained at 6 and 24 hours.

**Example 10: FAA beads with benzylated polyethylene imine shell**

The shell was applied on the FAA core by the process of solvent coacervation. The shell, Ben(84)-PEI, was dissolved in methanol and water mixture (3:1) at pH of 4.5. The shell and core were mixed for 5 minutes and methanol was removed by rotovap (40 minutes), isolated, washed, and dried.

Binding was measured under following conditions:

Donor solutions: 50 mM KCl and 50 mM $MgCl_2$
Bead concentration: 4 mg/ml
Duration: 6 hours

The potassium binding was calculated from actual magnesium uptake and overall binding capacity of polymer which was 5.74 meq/gm. The results are shown in Figure 10. Increasing the ratio of shell/core caused a decrease in magnesium binding which indicates an increase in potassium binding.

**Reference Example 11: Coating by controlled precipitation induced by pH change**

The shell comprised of Benzylated PEI, Ben (~20%); and Ben (~40%) on a Dowex(K) core. Binding was measured in 50 mM KCl and 50 mM $MgCl_2$.

Figure 11 shows the results of the binding experiments. Controlled precipitation method for 40% benzylated PEI shows better coating and this combination of coating method and materials gives higher binding selectivity.

**Reference Example 12: Membrane screening of Shell Polymers**

Shell polymers were screened by coating a flat membrane via solvent casting and using the coated membrane as the barrier in a diffusion cell, as depicted in Figure 15. Donor solution was 50mM 2-[N-morpholino] ethane sulfonic acid (MES) buffer at pH6.5 with 50mM $K^+$ and $Mg^{2+}$. Permeability coefficient was calculated as described in Example 4 above. Cross-linked B-PEI was tested using this method. B-PEI (35mol%) was cross-linked with 1, 4-butanediol diacrylate. The cross-linker was reacted on the top of dried B-PEI for 4 hours. The screening was performed in 50 mM KCl and 50 mM MgCl2 in 50 mM MES buffer. Cross-linker (diacrylate) reacted with B-PEI (35 mol%) membrane. As shown in Figure 13, addition of the cross-linker reduced permeability coefficient and also showed good selectivity.

Blends of Eudragit RL 100 and RS 100 were also evaluated using the method of Figure 12. The results are shown in Figure 14. Adding RS100 into RL100 can reduce the permeability and the permselectivity stays in the same range. Membranes with more than 50wt% of RS100 lost selectivity ([$K^+$] in the same scale, but [$Mg^{2+}$] much higher than other composites).

**Example 13: Effects of bile acids on $K^+$ binding**

Dowex(Li) (~100 μm) was first coated with PEI aqueous solution. The supernatant was removed and the coat was further crosslinked with 1,2-Bis-(2-iodoethoxy)-ethane (BIEE). Binding was measured in 50 mM KCl and 50 mM of MgCl2, MES buffer, pH 6.5. Bile acids extract used was 2 mg/ml (bile extract porcine with 60% bile acids and 40%

unknowns, i.e., free fatty acids, phospholipids, etc.). Time: 6 and 24 hrs and Bead content: 4 mg/ml. Results are shown in Figures 15A and 15B. Enhanced performance of the shell was observed in the presence of bile acids, fatty acids, and lipids.

**Example 13: Synthesis of methyl 2-fluoroacrylate beads**

All chemicals were purchased from commercial sources and used as received, except as noted. Reactions were carried out under nitrogen. The monomers used were methyl 2-fluoroacrylate (MeFA); crosslinkers were divinylbenzene (DVB); initiator: azobisisobutyronitrile (AIBN) and lauroyl peroxide (LPO); suspension stabilizer polyvinylalcohol (PVA) - MW 85,000-146,000, 87-89% hydrolyzed; and salt: sodium chloride (NaCl). MeFA and DVB were vacuum distilled.

*General procedure for synthesis of MeFA beads:*

To a 3-neck Morton-type flask equipped with a mechanical stirrer, a water condenser and a rubber septum were charged with an aqueous solution containing PVA (and NaCl in some cases). The solution was stirred and purged with nitrogen for 20 min. An organic solution containing MeFA, DVB and an initiator was added. The mixture was stirred at room temperature for 20 min, and heated in a 70-80°C oil bath for 2-6 hrs. The reaction mixture was cooled down to room temperature and the white solid was washed with water. The solid was examined by microscope and/or Malvern Master Sizer. The solid was either isolated by freeze-drying or used directly in the next step (hydrolysis reaction).

*General procedure for hydrolysis of MeFA beads to produce FAA beads:*

MeFA beads were suspended in 10 wt% NaOH (or KOH) aqueous solution at a concentration of 10 wt%. The mixture was heated in a 90°C oil bath for 20 hrs, and then allowed to cool down to room temperature. Solid was washed with water and 4M HCl and then freeze-dried.

*Synthesis of MeFA beads with no NaCl in aqueous phase and AIBN as initiator:*

To a 250mL 3-neck Morton-type flask equipped with a mechanical stirrer, a water condenser and a rubber septum were charged 75gm aqueous solution containing 1wt% PVA. The solution was stirred at 605 rpm and purged with nitrogen for 20 min. An organic solution containing MeFA (13.5g), DVB (1.5g) and AIBN (0.075g) was added. The mixture was stirred at room temperature for 20 min and heated in a 70°C oil bath for 6 hrs. The reaction mixture was cooled down to room temperature, and the white solid was washed with water. Large irregular particles (~1 mm) were observed under microscope

*Synthesis of MeFA beads with NaCl in aqueous phase and AIBN as initiator:*

To a 250mL 3-neck Morton-type flask equipped with a mechanical stirrer, a water condenser and a rubber septum were charged 75g aqueous solution containing 2wt% PVA and 3.75wt% NaCl. The solution was stirred at 502 rpm and purged with nitrogen for 20 min. An organic solution containing MeFA (13.5g), DVB (1.5g) and AIBN (0.075g) was added. The mixture was stirred at room temperature for 20 min, and heated in a 70°C oil bath for 6 hrs. The reaction mixture was cooled down to room temperature and the white solid was washed with water. Spherical beads (~90um) and some large gel particles were observed under microscope

*Synthesis of MeFA beads with no NaCl in aqueous phase and LPO as initiator:*

To a 250mL 3-neck Morton-type flask equipped with a mechanical stirrer, a water condenser and a rubber septum were charged 75g aqueous solution containing 2wt% PVA. The solution was stirred at 503rpm and purged with nitrogen for 20 min. An organic solution containing MeFA (13.5g), DVB (1.5g) and LPO (0.15g) was added. The mixture was stirred at room temperature for 20 min and heated in a 70°C oil bath for 2 hrs. The reaction mixture was cooled down to room temperature, and solid was washed with water, and freeze-dried. A white powder (11.85g) was obtained. Large irregular particles (0.5-1 mm) of aggregated beads were observed under microscope.

*Synthesis of MeFA beads with NaCl in aqueous phase and LPO as initiator:*

To a 1000mL 3-neck Morton-type flask equipped with a mechanical stirrer, a water condenser and a rubber septum were charged 300g aqueous solution containing 1wt% PVA and 3.75wt% NaCl. The solution was stirred at 307rpm and purged with nitrogen for 20 min. An organic solution containing MeFA (54g), DVB (6g) and LPO (0.6g) was

added. The mixture was stirred at room temperature for 20 min and heated in a 70°C oil bath for 4 hrs. The reaction mixture was cooled down to room temperature, solid was washed with water, and freeze-dried. A white powder (56g) was obtained. Spherical beads (~100μm) were observed under microscope.

**Example 14: In vivo efficacy of fluoroacrylate (FAA) polymer-$NH_4$ form compared to Kayexalate (Polystyrene Sulfonate)**

40 male rats were acclimated for three days on Harlan Teklad Diet TD.04498, whereupon they were randomly assigned to four groups of ten rats. The four groups were then fed for a further four days an admixture of Harlan Teklad Diet TD.04498 with test or control articles according to Table 18.

TABLE 18

| Group | Number of Animals | Treatment Groups | Test Article Concentration in Diet (g/kg) | Dose levels (% diet w/w) |
|---|---|---|---|---|
| 1 | 10 | Cellulose Control | 20 | 2% |
| 2 | 10 | Kayexalate: $NH_4^+$-form | **21.5** | **2.15%** |
| 3 | 10 | FAA polymer:$NH_4^+$-form | **23** | **2.3%** |
| 4 | 10 | FAA polymer:$NH_4^+$-form | **11.5** | **1.15%** |

2.15% Kayexalate: $NH_4^+$-form corresponds to 2% Kayexalate: $H^+$-form and 2.3% FAA polymer:$NH_4^+$-form corresponds to 2% FAA polymer:$H^+$-form. The binding capacity values reported below correspond to the $H^+$-form polymers. The FAA-polymer used in this *in vivo* study was synthesized using the same procedure as shown in Table 11, for polymer number 100982A1, and the material was further ion exchanged with ammonium ions.

Feces were collected from each rat and pooled each 24hrs. Feces were lyophilized and dry weights per rat per day were recorded. Fecal cations were extracted in 1 M HCl overnight and measured using Ion Chromatography. The total moles of each cation (Sodium, Ammonium, Potassium, Magnesium and Calcium) excreted into the feces of each rat per day was calculated.

It was determined that the effect of the polymers on fecal cations reached equilibrium after two days of treatment. The data for the third and fourth days were pooled and are shown in Figure 17. A statistical analysis of the data from the third and fourth days of treatment indicates that FAA polymer: $NH_4^+$-form binds significantly more Sodium, Ammonium, Potassium and Calcium than does Kayexalate.

The amount of each cation (in mEq) bound per gram of $H^+$-form polymer was calculated based on the dietary intake of polymer and the difference between the amount of cation in the feces of control animals versus the amount of cation in the feces of test animals on diets containing 2% test articles. The calculated *in vivo* binding capacities for Kayexalate and FAA polymer:$NH_4^+$-form are shown in Table 19.

TABLE 19: mEg cations bound *in vivo* per g resin (when present at 2% in diet)

| | **Na** | **$NH_4$** | **K** | **Mg** | **Ca** | **Total mEq** |
|---|---|---|---|---|---|---|
| **Kayexalate** | 1.09 | 0.41 | 0.24 | 0.66 | 0.46 | 2.87 |
| FAA polymer:$NH_4^+$-form | 2.11 | 1.10 | 0.44 | 1.13 | 1.30 | 6.07 |

While preferred embodiments of the present invention have been shown and described herein, it will be obvious to those skilled In the art that such enbodiments are provided by way of example only. Numerous variations, changes, and substitutions will now occur to those skilled in the art without departing from the Invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

**1.** A composition for use in removing potassium from a patient, the composition comprising
a cation exchange polymer comprising acid groups selected from the group consisting of sulfonic ($-SO_3^-$), sulfuric ($-OSO_3^-$), carboxylic ($-CO_2^-$), phosphonic ($-PO_3^{2-}$), phosphoric ($-OPO_3^{2-}$), and sulfamate ($-NHSO_3^-$) groups, and an electron-withdrawing substituent located adjacent to the acid group, wherein the electron-withdrawing substituent

is selected from the group consisting of a hydroxyl group, an ether group, an ester group, and a halide atom wherein said polymer is obtainable by a heterogeneous polymerization process; and
one or more pharmaceutically acceptable excipients.

**2.** A composition for use in removing potassium from a patient according to claim 1 comprising:

a cation exchange polymer comprising a monomeric unit having an acid group selected from the group consisting of sulfonic ($-SO_3^-$), sulfuric ($-OSO_3^-$), carboxylic ($-CO_2^-$), phosphonic ($-PO_3^{2-}$), phosphoric ($-OPO_3^{2-}$), and sulfamate ($- NHSO_3^-$) groups, and
an electron-withdrawing substituent located adjacent to the acid group, wherein the electron-withdrawing substituent is selected from the group consisting of a hydroxyl group, an ether group, an ester group, and a halide atom, wherein said polymer is obtainable by a heterogeneous polymerization process; and
a pharmaceutically acceptable excipient.

**3.** The composition of claim 1 or 2 wherein the use includes treatment of hyperkalemia resulting from renal insufficiency caused by treatment with a potassium-sparing diuretic, an angiotensin-converting enzyme inhibitor, an angiotensin receptor blocker, a non-steroidal anti-inflammatory drug, heparin, or trimethoprim.

**4.** The composition of claim 1 or 2 wherein the composition further comprises a potassium-sparing diuretic, an angiotensin-converting enzyme inhibitor, a non-steroidal anti-inflammatory drug, heparin, or trimethoprim.

**5.** The composition of any one of claims 1, 2 or 4 wherein the heterogeneous polymerization process is a suspension polymerization.

**6.** The composition of any one of claims 1, 2 or 5 wherein the polymer comprises an alpha-fluoroacrylate polymer.

**7.** The composition of any one of claims 1, 2 or 5 wherein the polymer comprises a methyl 2-fluoroacrylate ester.

**8.** The composition of claim 6 or 7 wherein the polymer is produced from a suspension polymerization mixture comprising a free radical initiator.

**9.** The composition of claim 8 wherein the polymer is produced from a suspension polymerization mixture further comprising water-soluble salt.

**10.** The composition of any one of claims 1 to 9 wherein the polymer comprises a cationic counterion of $Ca^{2+}$, $H^+$, $NH^{4+}$, $Na^+$, or a combination thereof.

**11.** The composition of any one of claims 1 to 10 wherein the acid group is in its protonated, ionized or anhydride form.

**12.** The composition of any one of claims 1 to 11 wherein the polymer is used without a shell and the polymer is other than sodium polystyrene sulfonate or ammonium polystyrene sulfonate.

**13.** The composition of any one of claims 1 to 12 wherein the polymer is a crosslinked polymer.

**14.** The composition of any one of claims 1 to 13 wherein the polymer is an 2-fluoroacrylic acid polymer crosslinked with divinylbenzene, ethylene bisacrylamide, N,N'- bis(vinylsulfonylacetyl) ethylene diamine, 1,3-bis(vinylsulfonyl) 2-propanol, vinylsulfone, N,N'-methylenebisacrylamide polyvinyl ether, polyallylether, or a combination thereof.

**15.** The composition of any one of claims 6 to 9 wherein the polymer is in the form of a bead.

**16.** Use of the composition comprising
a cation exchange polymer comprising acid groups selected from the group consisting of sulfonic ($-SO_3^-$), sulfuric ($-OSO_3^-$), carboxylic ($-CO_2^-$), phosphonic ($-PO_3^{2-}$), phosphoric ($-OPO_3^{2-}$), and sulfamate ($- NHSO_3^-$) groups, and an electron-withdrawing substituent located adjacent to the acid group, wherein the electron-withdrawing substituent is selected from the group consisting of a hydroxyl group, an ether group, an ester group, and a halide atom, wherein said polymer is obtainable by a heterogeneous polymerization process; and
one or more pharmaceutically acceptable excipients;
for the manufacture of a medicament for the treatment and/or prophylaxis of hyperkalemia.

## Patentansprüche

1. Eine Zusammensetzung zur Verwendung zum Entfernen von Kalium aus einem Patienten, die Zusammensetzung umfassend
ein Kationenaustauschpolymer enthaltend Säuregruppen ausgewählt aus der Gruppe bestehend aus Sulfonsäure- ($-SO_3^-$), Schwefelsäure- ($-OSO_3^-$), Carboxylsäure- ($-CO_2^-$), Phosphonsäure- ($-PO_3^{2-}$), Phosphorsäure- ($-OPO_3^{2-}$), und Sulfamat- ($-NHSO_3^-$) Gruppen, und einen zu der Säuregruppe benachbart angeordneten Elektronen-entziehenden Substituenten, wobei der Elektronen-entziehende Substituent ausgewählt ist aus der Gruppe bestehend aus einer Hydroxylgruppe, einer Ethergruppe, einer Estergruppe, und einem Halogenidatom,
wobei das besagte Polymer durch einen heterogenen Polymerisationsprozess erhältlich ist; und
einen oder mehrere pharmazeutisch akzeptable Hilfsstoffe.

2. Eine Zusammensetzung zur Verwendung zum Entfernen von Kalium aus einem Patienten nach Anspruch 1 umfassend:

   ein Kationenaustauschpolymer enthaltend eine monomere Einheit, die eine Säuregruppe ausgewählt aus der Gruppe bestehend aus Sulfonsäure- ($-SO_3^-$), Schwefelsäure- ($-OSO_3^-$), Carboxylsäure- ($-CO_2^-$), Phosphonsäure- ($-PO_3^{2-}$), Phosphorsäure- ($-OPO_3^{2-}$), und Sulfamat- ($-NHSO_3^-$) Gruppen aufweist, und
   einen zu der Säuregruppe benachbart angeordneten Elektronen-entziehenden Substituenten, worin der Elektronen-entziehende Substituent ausgewählt ist aus der Gruppe bestehend aus einer Hydroxylgruppe, einer Ethergruppe, einer Estergruppe, und einem Halogenidatom, worin das besagte Polymer durch einen heterogenen Polymerisationsprozess erhältlich ist; und
   einen pharmazeutisch akzeptablen Hilfsstoff.

3. Die Zusammensetzung nach Anspruch 1 oder 2, worin die Verwendung die Behandlung von Hyperkaliämie einschließt, die aus einer, durch Behandlung mit einem Kalium-sparenden Diuretikum, einem Angiotensin-konvertierenden Enzyminhibitor, einem Angiotensin-Rezeptorblocker, einer nicht-steroidalen antiinflammatorischen Arznei, Heparin, oder Trimethoprim verursachten, renalen Insuffizienz resultiert.

4. Die Zusammensetzung nach Anspruch 1 oder 2, wobei die Zusammensetzung weiterhin ein Kalium-sparendes Diuretikum, einen Angiotensin-konvertierenden Enzyminhibitor, eine nicht-steroidale anti-inflammatorische Arznei, Heparin, oder Trimethoprim umfasst.

5. Die Zusammensetzung nach einem der Ansprüche 1, 2 oder 4, wobei der heterogene Polymerisationsprozess eine Suspensionspolymerisation ist.

6. Die Zusammensetzung nach einem der Ansprüche 1, 2 oder 5, wobei das Polymer ein alpha-Fluoracrylatpolymer umfasst.

7. Die Zusammensetzung nach einem der Ansprüche 1, 2 oder 5, wobei das Polymer einen Methyl-2-Fluoracrylatester umfasst.

8. Die Zusammensetzung nach Anspruch 6 oder 7, wobei das Polymer aus einer, einen freien Radikalinitiator enthaltenden Suspensionspolymerisation-Mischung hergestellt ist.

9. Die Zusammensetzung nach Anspruch 8, wobei das Polymer aus einer, weiterhin ein wasserlösliches Salz enthaltende, Suspensionspolymerisation-Mischung hergestellt ist.

10. Die Zusammensetzung nach einem der Ansprüche 1 bis 9, wobei das Polymer ein kationisches Gegenion von $Ca^{2+}$, $H^+$, $NH^{4+}$, $Na^+$, oder eine Kombination davon umfasst.

11. Die Zusammensetzung nach einem der Ansprüche 1 bis 10, wobei die Säuregruppe in ihrer protonierten, ionisierten oder Anhydrid-Form vorliegt.

12. Die Zusammensetzung nach einem der Ansprüche 1 bis 11, wobei das Polymer ohne Hülle verwendet wird und das Polymer ein anderes ist als Natriumpolystyrolsulfonat oder Ammoniumpolystyrolsulfonat.

13. Die Zusammensetzung nach einem der Ansprüche 1 bis 12, wobei das Polymer ein quervernetztes Polymer ist.

**14.** Die Zusammensetzung nach einem der Ansprüche 1 bis 13, wobei das Polymer ein 2-Fluoroacrylsäurepolymer ist, das mit Divinylbenzol, Ethylen-bis-acrylamid, N,N'- bis(Vinylsulfonylacetyl)-ethylendiamin, 1,3-bis(Vinylsulfonyl)-2-propanol, Vinylsulfon, N,N'-Methylen-bis-acrylamid-polyvinylether, Polyallylether, oder einer Kombination davon, quervernetzt ist.

**15.** Die Zusammensetzung nach einem der Ansprüche 6 bis 9, wobei das Polymer in der Form eines Kügelchens vorliegt.

**16.** Verwendung der Zusammensetzung umfassend
ein Kationenaustauschpolymer enthaltend Säuregruppen ausgewählt aus der Gruppe bestehend aus Sulfonsäure- ($-SO_3^-$), Schwefelsäure- ($-OSO_3^-$), Carboxylsäure-($-CO_2^-$), Phosphonsäure- ($-PO_3^{2-}$), Phosphorsäure- ($-OPO_3^{2-}$), und Sulfamat- ($-NHSO_3^-$) Gruppen, und einen zu der Säuregruppe benachbart angeordneten Elektronen-entziehenden Substituenten, wobei der Elektronen-entziehende Substituent ausgewählt ist aus der Gruppe bestehend aus einer Hydroxylgruppe, einer Ethergruppe, einer Estergruppe, und einem Halogenidatom,
wobei das besagte Polymer durch einen heterogenen Polymerisationsprozess erhältlich ist;
und
einen oder mehrere pharmazeutisch akzeptable Hilfsstoffe;
zur Herstellung eines Medikamentes zur Behandlung und/oder Prophylaxe von Hyperkaliämie.

## Revendications

**1.** Composition destinée à une utilisation pour éliminer du potassium d'un patient, la composition comprenant
un polymère échangeur de cations comprenant des groupes acides choisis parmi le groupe consistant en des groupes sulfonique ($-SO_3^-$), sulfurique ($-OSO_3^-$), carboxylique ($-CO_2^-$), phosphonique ($-PO_3^{2-}$), phosphorique ($-OPO_3^{2-}$), et sulfamate ($-NHSO_3^-$), et un substituant de retrait d'électrons situé de façon adjacente au groupe acide, dans laquelle le substituant de retrait d'électrons est choisi parmi le groupe consistant en un groupe hydroxyle, un groupe éther, un groupe ester, et un atome d'halogénure
dans laquelle ledit polymère peut être obtenu par un processus de polymérisation hétérogène ; et
un ou plusieurs excipient(s) pharmaceutiquement acceptable(s).

**2.** Composition destinée à une utilisation pour éliminer du potassium d'un patient selon la revendication 1 comprenant :

un polymère échangeur de cations comprenant une unité monomérique ayant un groupe acide choisi parmi le groupe consistant en des groupes sulfonique ($-SO_3^-$), sulfurique ($-OSO_3^-$), carboxylique ($-CO_2^-$) phosphonique ($-PO_3^{2-}$), phosphorique ($-OPO_3^{2-}$), et sulfamate ($-NHSO_3^-$), et
un substituant de retrait d'électrons situé de façon adjacente au groupe acide, dans laquelle le substituant de retrait d'électrons est choisi parmi le groupe consistant en un groupe hydroxyle, un groupe éther, un groupe ester, et un atome d'halogénure, dans laquelle ledit polymère peut être obtenu par un processus de polymérisation hétérogène ; et
un excipient pharmaceutiquement acceptable.

**3.** Composition selon la revendication 1 ou 2 dans laquelle l'utilisation inclut un traitement d'une hyperkaliémie résultant d'une insuffisance rénale causée par un traitement avec un diurétique d'épargne potassique, un inhibiteur de l'enzyme de conversion de l'angiotensine, un bloqueur du récepteur de l'angiotensine, un médicament anti-inflammatoire non stéroïdien, l'héparine, ou le triméthoprime.

**4.** Composition selon la revendication 1 ou 2 dans laquelle la composition comprend en outre un diurétique d'épargne potassique, un inhibiteur de l'enzyme de conversion de l'angiotensine, un médicament antiinflammatoire non stéroïdien, de l'héparine, ou du triméthoprime.

**5.** Composition selon l'une quelconque des revendications 1, 2 ou 4 dans laquelle le processus de polymérisation hétérogène est une polymérisation en suspension.

**6.** Composition selon l'une quelconque des revendications 1, 2 ou 5 dans laquelle le polymère comprend un polymère d'alpha-fluoroacrylate.

**7.** Composition selon l'une quelconque des revendications 1, 2 ou 5 dans laquelle le polymère comprend un ester de 2-fluoroacrylate de méthyle.

**8.** Composition selon la revendication 6 ou 7 dans laquelle le polymère est produit à partir d'un mélange de polymérisation en suspension comprenant un initiateur de radicaux libres.

**9.** Composition selon la revendication 8 dans laquelle le polymère est produit à partir d'un mélange de polymérisation en suspension comprenant en outre un sel soluble dans l'eau.

**10.** Composition selon l'une quelconque des revendications 1 à 9 dans laquelle le polymère comprend un contre-ion cationique de $Ca^{2+}$, $H^+$, $NH^{4+}$, $Na^+$, ou d'une combinaison de ceux-ci.

**11.** Composition selon l'une quelconque des revendications 1 à 10 dans laquelle le groupe acide est sous sa forme protonée, ionisée ou anhydride.

**12.** Composition selon l'une quelconque des revendications 1 à 11 dans laquelle le polymère est utilisé sans enveloppe et le polymère est autre qu'un sulfonate de polystyrène de sodium ou un sulfonate de polystyrène d'ammonium.

**13.** Composition selon l'une quelconque des revendications 1 à 12 dans laquelle le polymère est un polymère réticulé.

**14.** Composition selon l'une quelconque des revendications 1 à 13 dans laquelle le polymère est un polymère d'acide 2-fluoroacrylique réticulé avec du divinylbenzène, du bisacrylamide d'éthylène, une N,N'-bis(vinylsulfonylacétyl) éthylène diamine, du 1,3-bis(vinylsulfonyl) 2-propanol, du vinylsulfone, un éther polyvinylique de N,N'-méthylènebisacrylamide, un éther polyallylique, ou une combinaison de ceux-ci.

**15.** Composition selon l'une quelconque des revendications 6 à 9 dans laquelle le polymère est sous la forme d'une bille.

**16.** Utilisation de la composition comprenant
un polymère échangeur de cations comprenant des groupes acides choisis parmi le groupe consistant en des groupes sulfonique ($-SO_3^-$), sulfurique ($-OSO_3^-$), carboxylique ($-CO_2^-$), phosphonique ($-PO_3^{2-}$), phosphorique ($-OPO_3^{2-}$), et sulfamate ($-NHSO_3^-$), et un substituant de retrait d'électrons situé de façon adjacente au groupe acide, dans laquelle le substituant de retrait d'électrons est choisi parmi le groupe consistant en un groupe hydroxyle, un groupe éther, un groupe ester, et un atome d'halogénure,
dans laquelle ledit polymère peut être obtenu par un processus de polymérisation hétérogène ; et
un ou plusieurs excipient(s) pharmaceutiquement acceptable(s) ;
pour la fabrication d'un médicament pour le traitement et/ou la prophylaxie d'une hyperkaliémie.

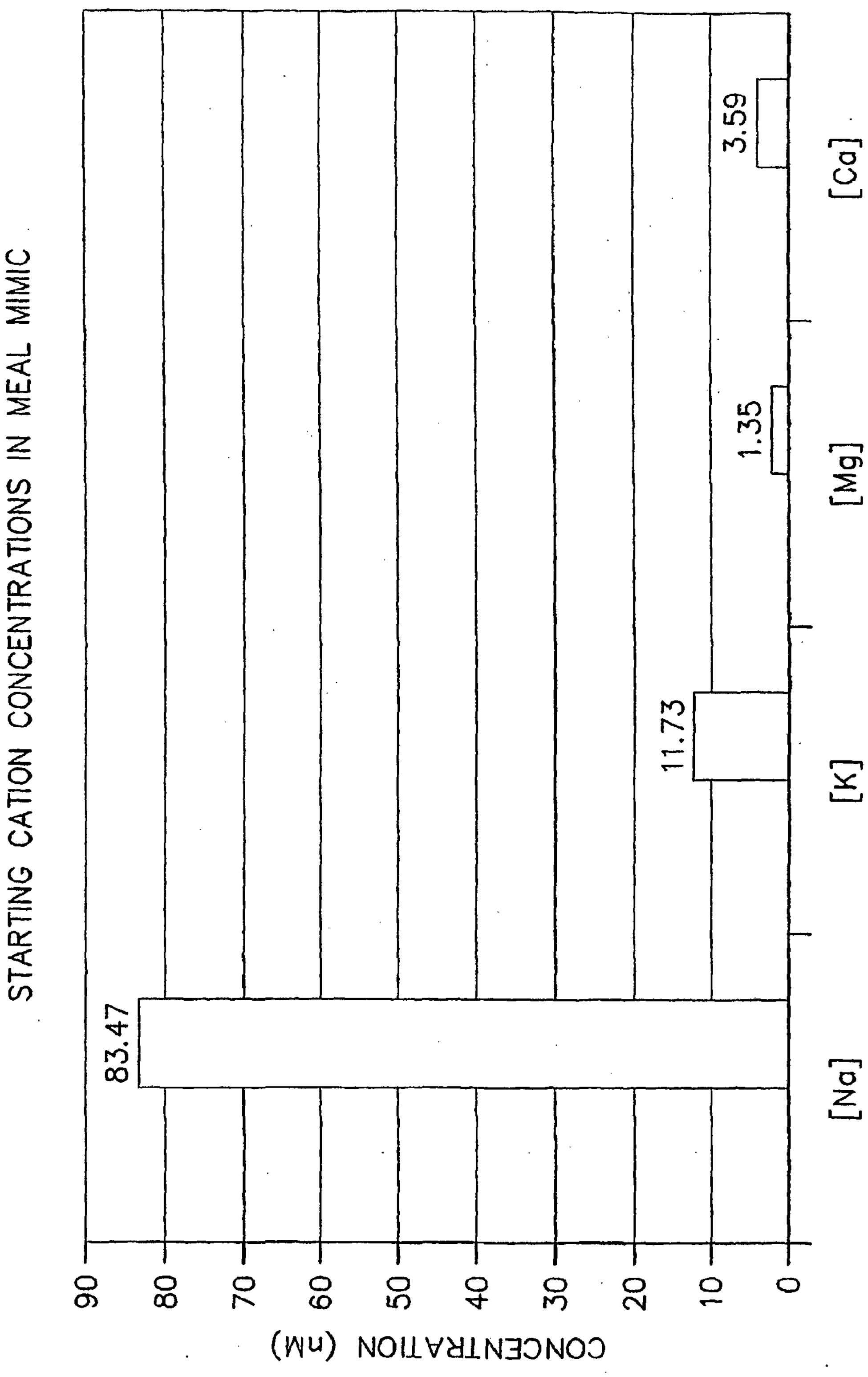
STARTING CATION CONCENTRATIONS IN MEAL MIMIC
CONCENTRATION (nM)
0
10
20
30
40
50
60
70
80
90
83.47
11.73
1.35
3.59
[Na]
[K]
[Mg]
[Ca]

FIG. 1

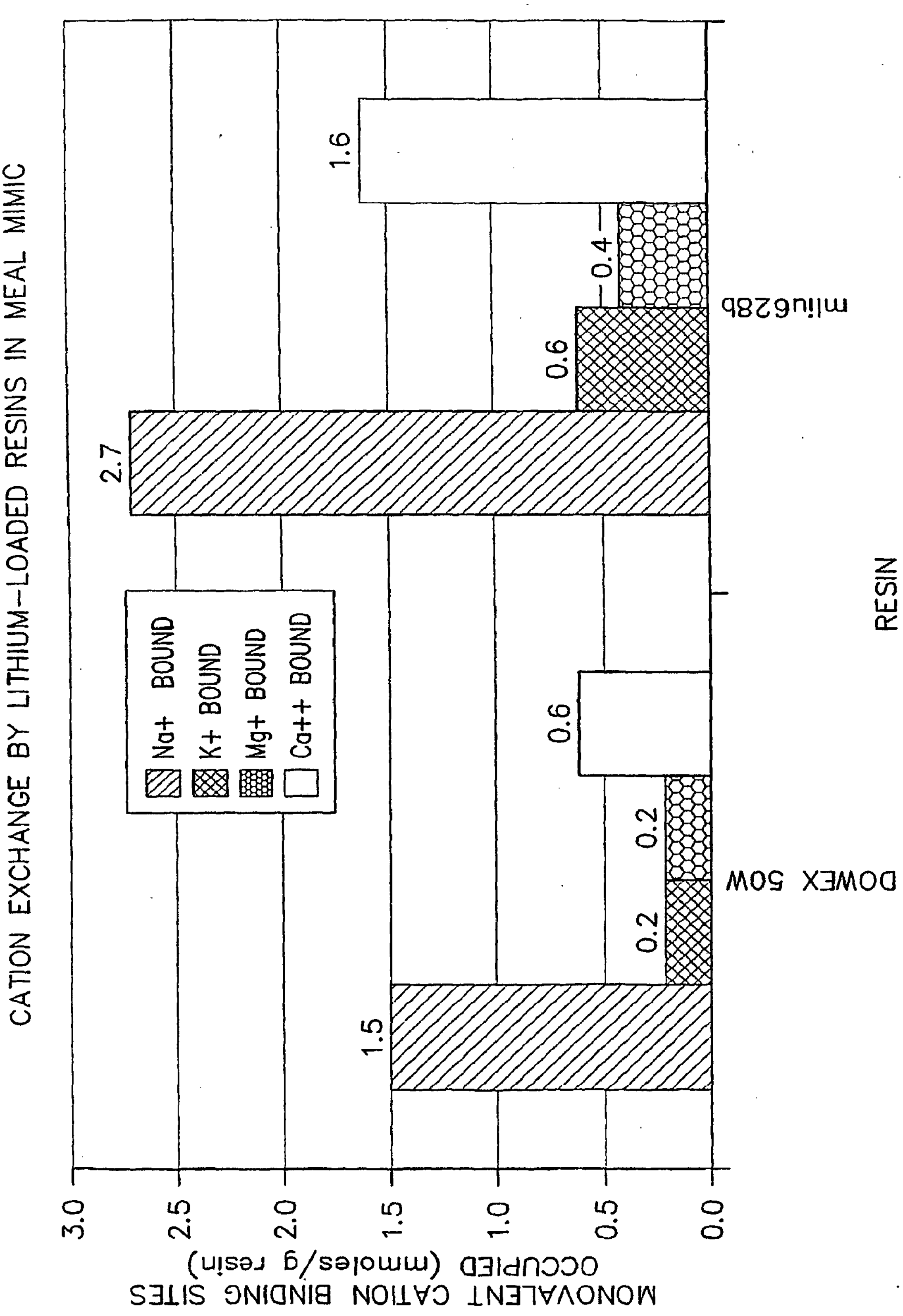
CATION EXCHANGE BY LITHIUM-LOADED RESINS IN MEAL MIMIC
MONOVALENT CATION BINDING SITES OCCUPIED (mmoles/g resin)
3.0
2.5
2.0
1.5
1.0
0.5
0.0
Na+ BOUND
K+ BOUND
Mg+ BOUND
Ca++ BOUND
1.5
0.2
0.2
0.6
2.7
0.6
0.4
1.6
DOWEX 50W
miIu628b
RESIN

FIG. 2

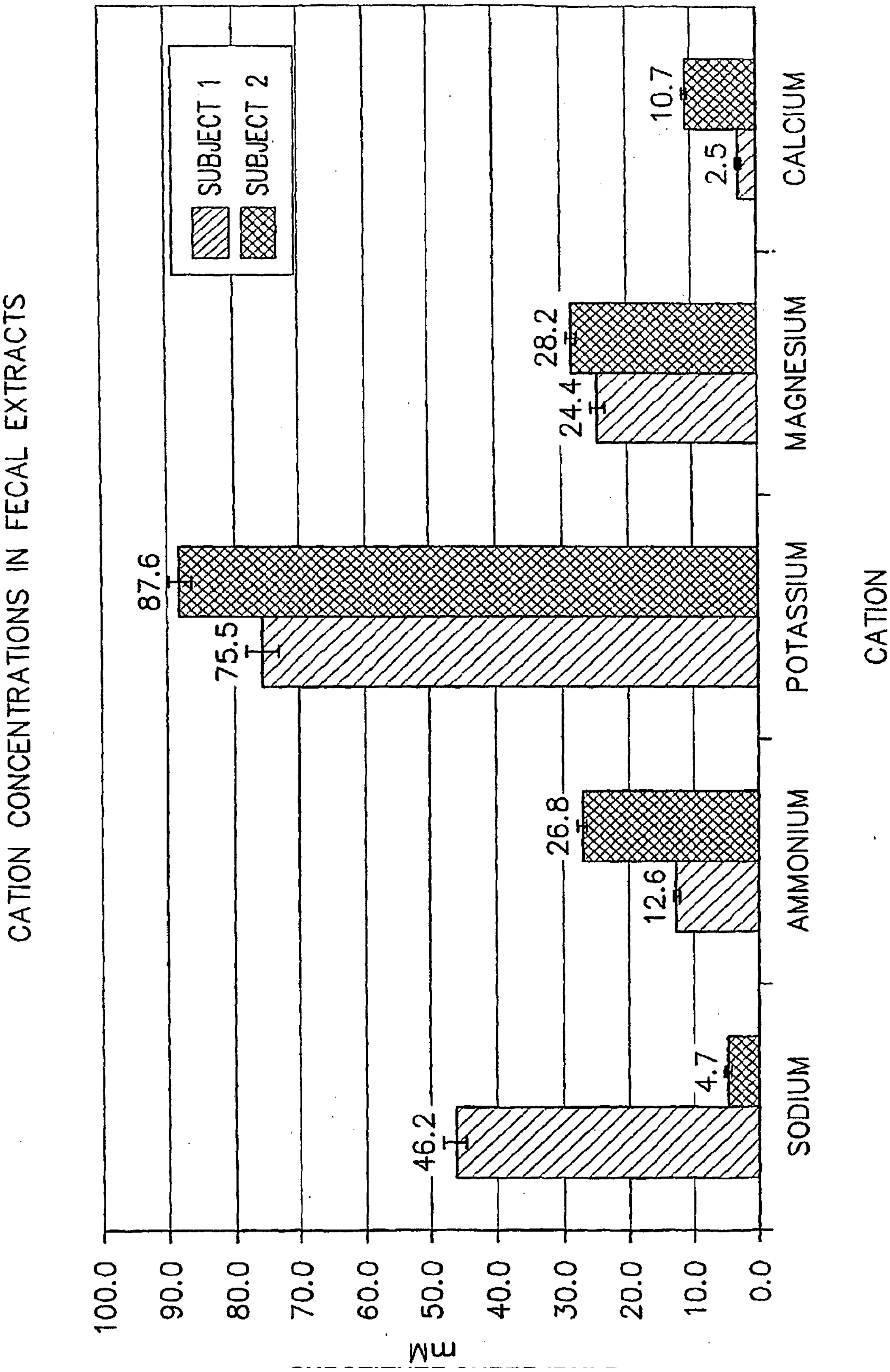
CATION CONCENTRATIONS IN FECAL EXTRACTS
SUBJECT 1
SUBJECT 2
mM
100.0
90.0
80.0
70.0
60.0
50.0
40.0
30.0
20.0
10.0
0.0
46.2
4.7
12.6
26.8
75.5
87.6
24.4
28.2
2.5
10.7
SODIUM
AMMONIUM
POTASSIUM
MAGNESIUM
CALCIUM
CATION

FIG. 3

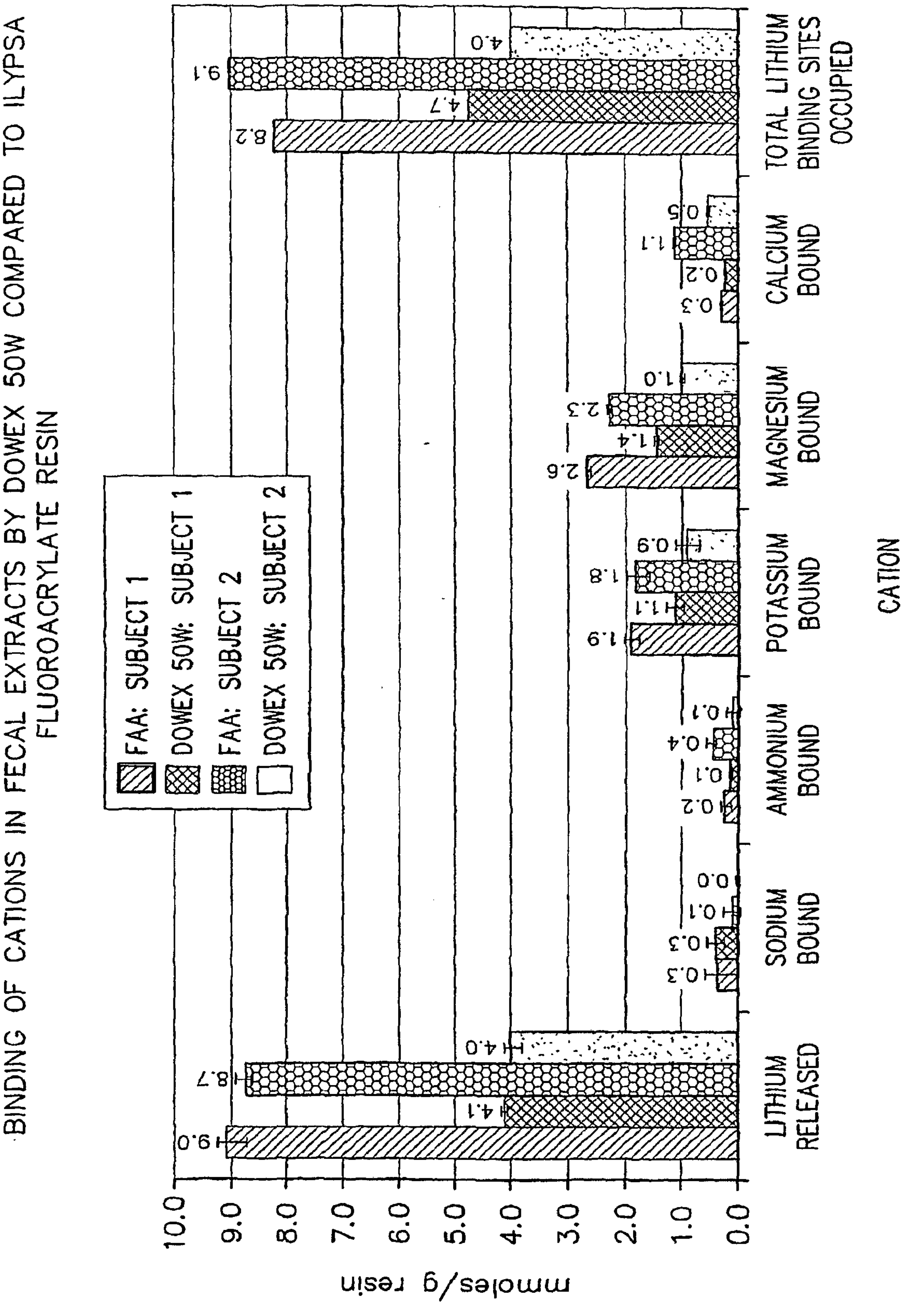
BINDING OF CATIONS IN FECAL EXTRACTS BY DOWEX 50W COMPARED TO ILYPSA FLUOROACRYLATE RESIN
FAA: SUBJECT 1
DOWEX 50W: SUBJECT 1
FAA: SUBJECT 2
DOWEX 50W: SUBJECT 2
mmoles/g resin
10.0
9.0
8.0
7.0
6.0
5.0
4.0
3.0
2.0
1.0
0.0
LITHIUM RELEASED
9.0
4.1
8.7
4.0
SODIUM BOUND
0.3
0.3
0.1
0.0
AMMONIUM BOUND
0.2
0.1
0.4
0.1
POTASSIUM BOUND
1.9
1.1
1.8
0.9
MAGNESIUM BOUND
2.6
1.4
2.3
1.0
CALCIUM BOUND
0.3
0.2
1.1
0.5
TOTAL LITHIUM BINDING SITES OCCUPIED
8.2
4.7
9.1
4.0
CATION

FIG. 4

FIG. 5

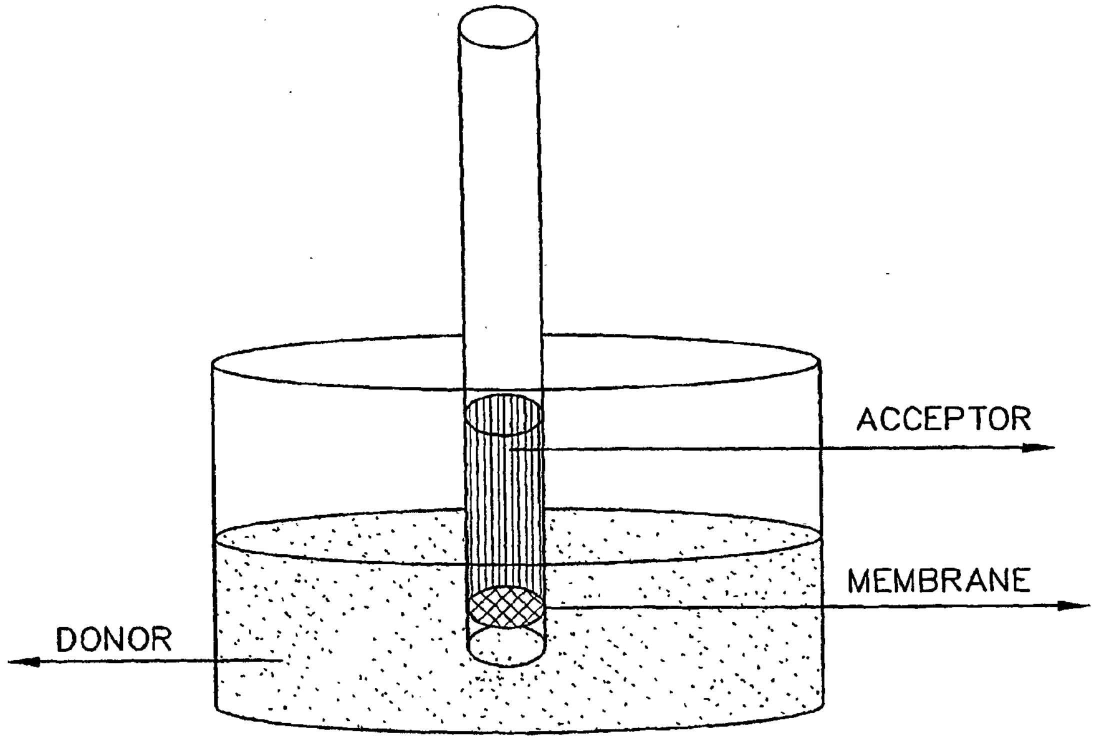

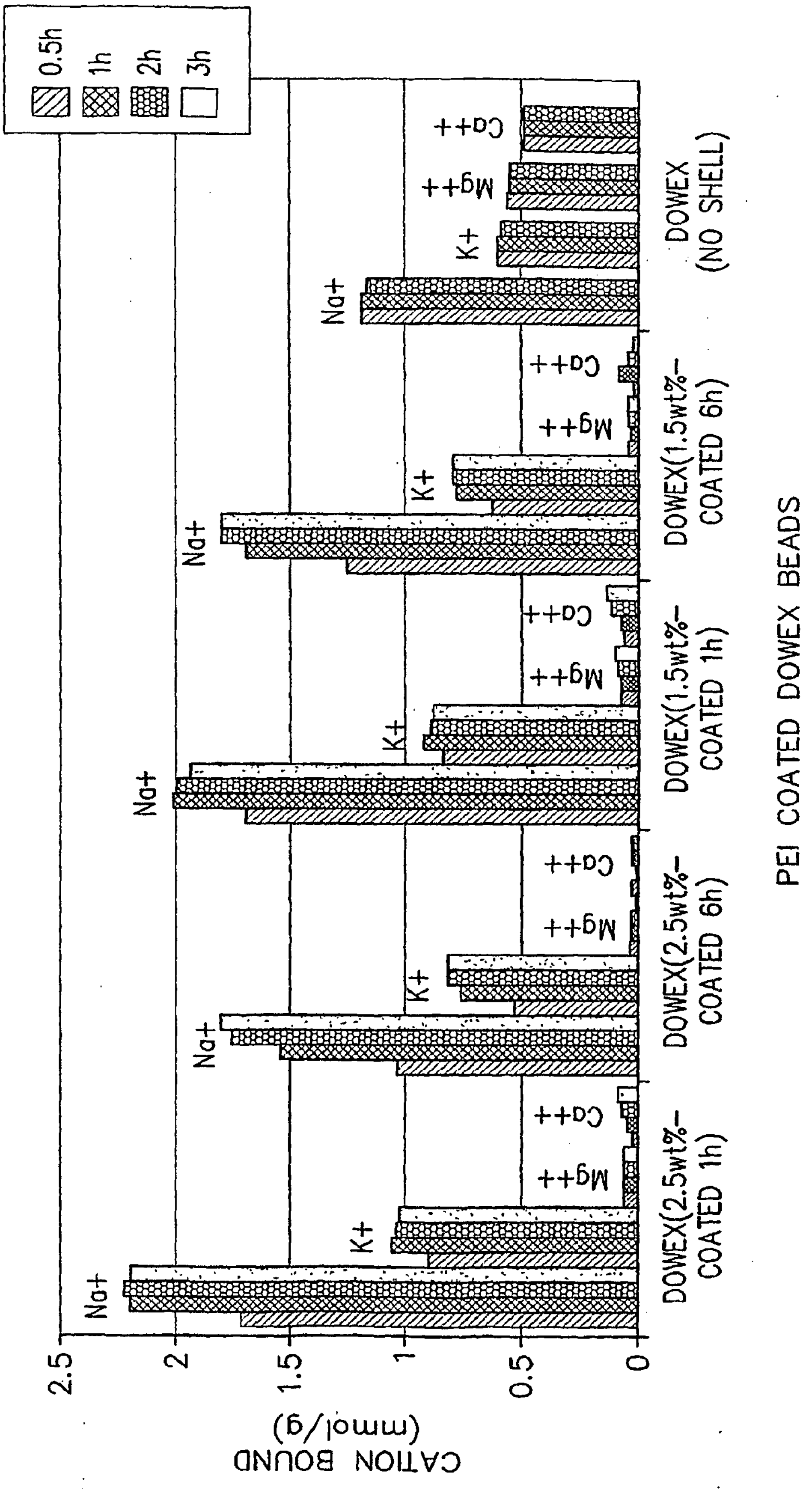
0.5h
1h
2h
3h
CATION BOUND
(mmol/g)
2.5
2
1.5
1
0.5
0
Na+
K+
Mg++
Ca++
DOWEX(2.5wt%-
COATED 1h)
DOWEX(2.5wt%-
COATED 6h)
DOWEX(1.5wt%-
COATED 1h)
DOWEX(1.5wt%-
COATED 6h)
DOWEX
(NO SHELL)
PEI COATED DOWEX BEADS

FIG. 6

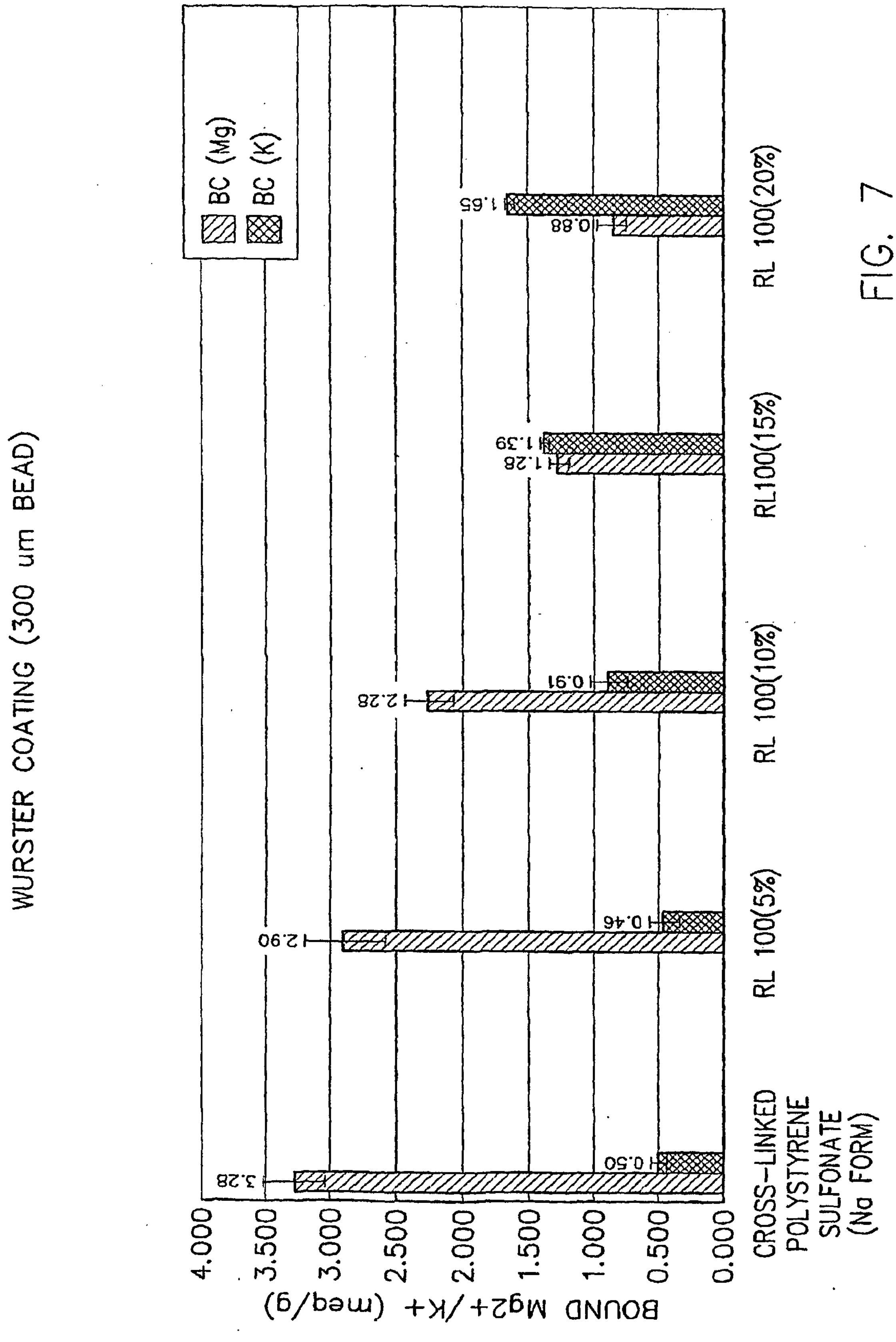
WURSTER COATING (300 um BEAD)
BOUND Mg2+/K+ (meq/g)
4.000
3.500
3.000
2.500
2.000
1.500
1.000
0.500
0.000
BC (Mg)
BC (K)
3.28
0.50
2.90
0.46
2.28
0.91
1.28
1.39
0.88
1.65
CROSS-LINKED POLYSTYRENE SULFONATE (Na FORM)
RL 100(5%)
RL 100(10%)
RL100(15%)
RL 100(20%)

FIG. 7

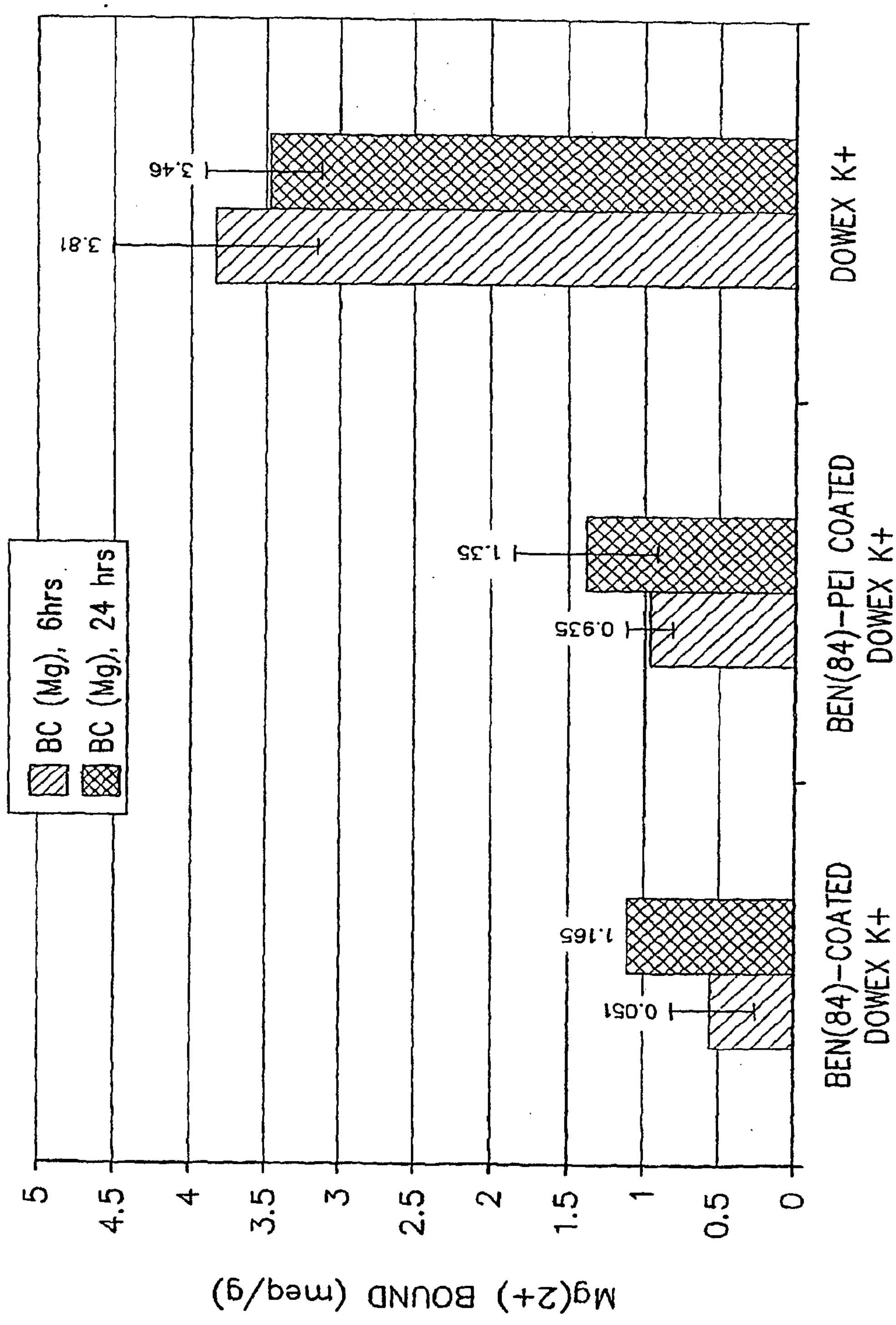
BC (Mg), 6hrs
BC (Mg), 24 hrs
3.81
3.46
1.35
0.935
1.165
0.051
BEN(84)–COATED DOWEX K+
BEN(84)–PEI COATED DOWEX K+
DOWEX K+
5
4.5
4
3.5
3
2.5
2
1.5
1
0.5
0
Mg(2+) BOUND (meq/g)

FIG. 8

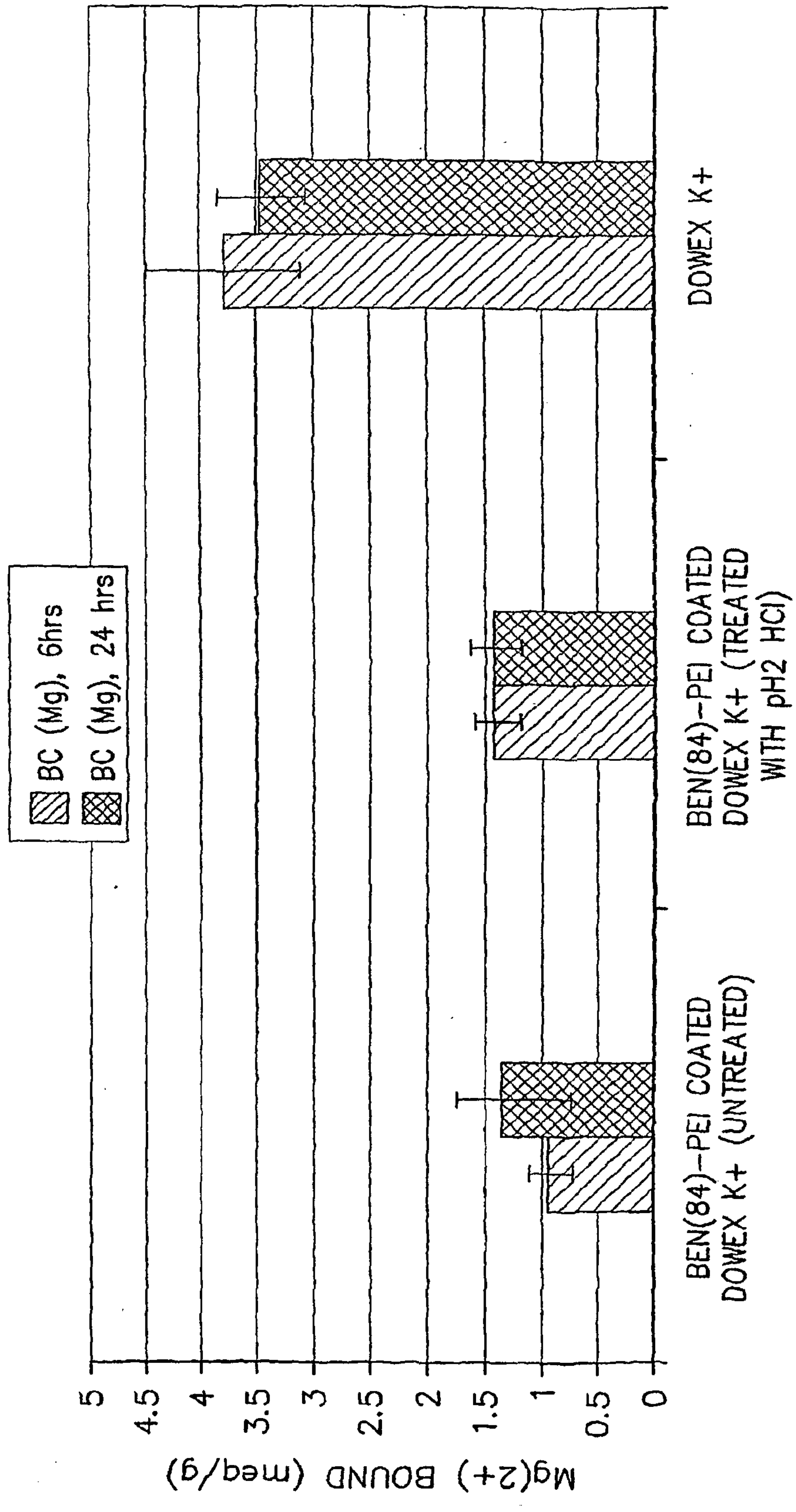
BC (Mg), 6hrs
BC (Mg), 24 hrs
Mg(2+) BOUND (meq/g)
0
0.5
1
1.5
2
2.5
3
3.5
4
4.5
5
BEN(84)-PEI COATED DOWEX K+ (UNTREATED)
BEN(84)-PEI COATED DOWEX K+ (TREATED WITH pH2 HCl)
DOWEX K+

FIG. 9

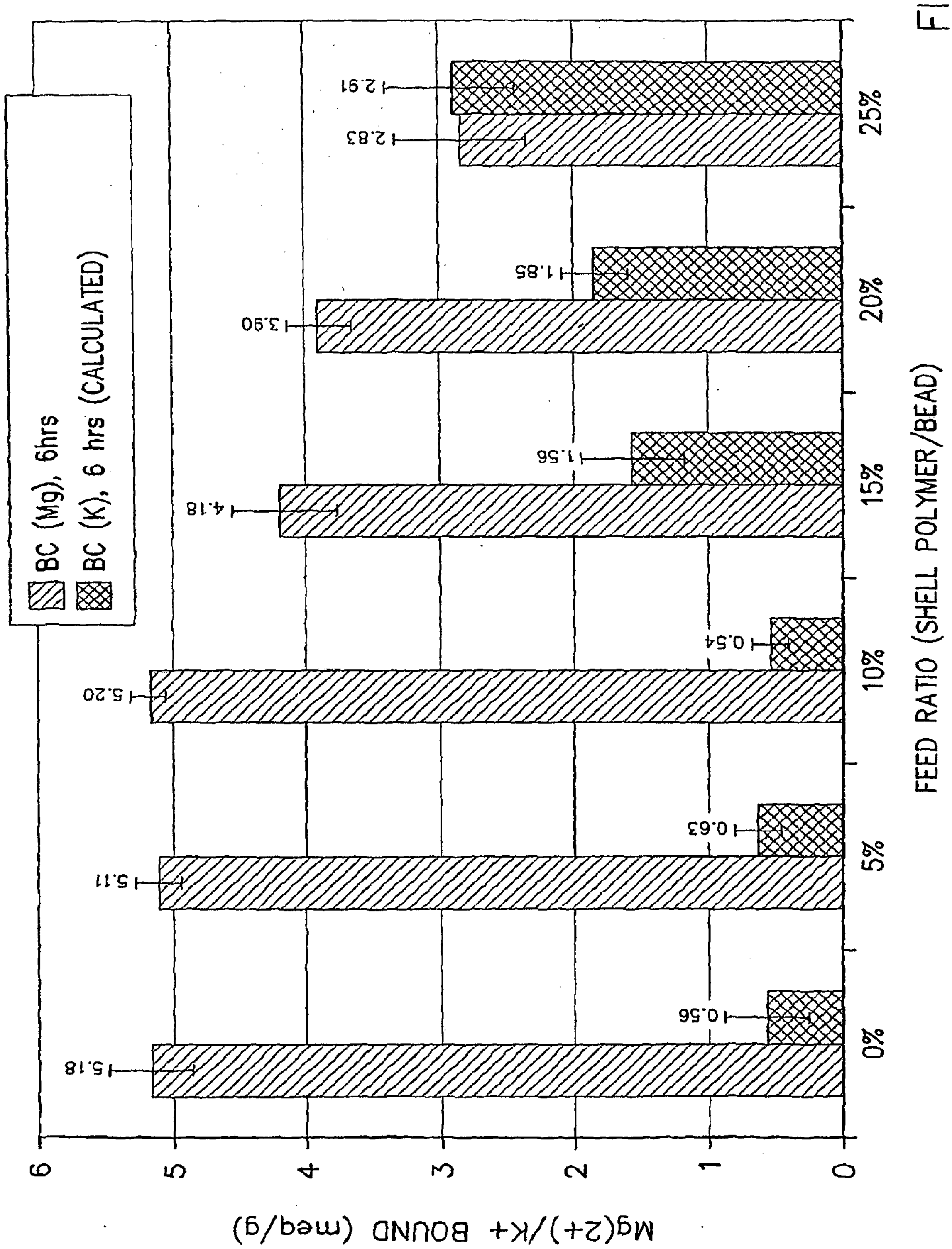
BC (Mg), 6hrs
BC (K), 6 hrs (CALCULATED)
Mg(2+)/K+ BOUND (meq/g)
0
1
2
3
4
5
6
FEED RATIO (SHELL POLYMER/BEAD)
0%
5%
10%
15%
20%
25%
5.18
0.56
5.11
0.63
5.20
0.54
4.18
1.56
3.90
1.85
2.83
2.91

FIG. 10

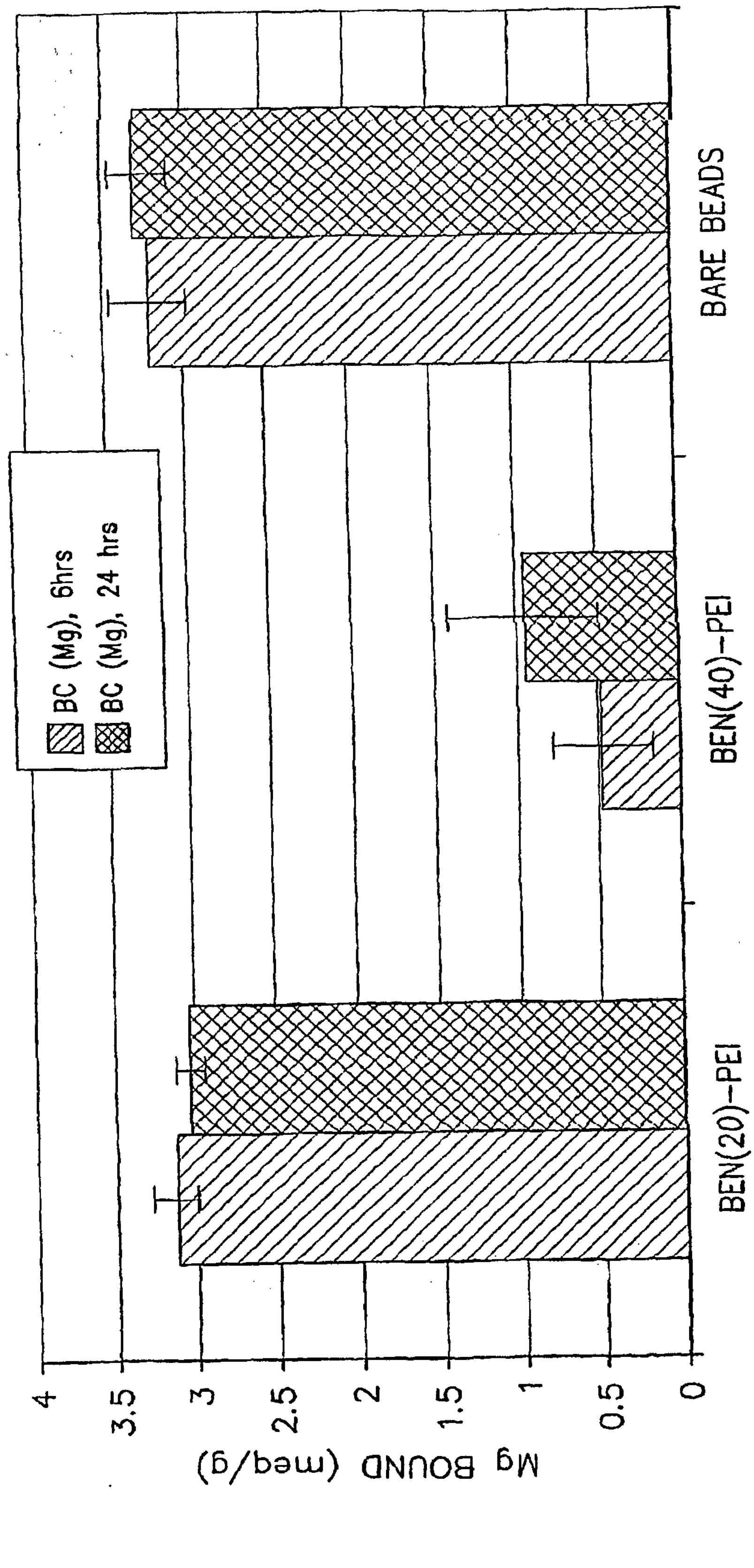
BC (Mg), 6hrs
BC (Mg), 24 hrs
Mg BOUND (meq/g)
0
0.5
1
1.5
2
2.5
3
3.5
4
BEN(20)–PEI
BEN(40)–PEI
BARE BEADS

FIG. 11

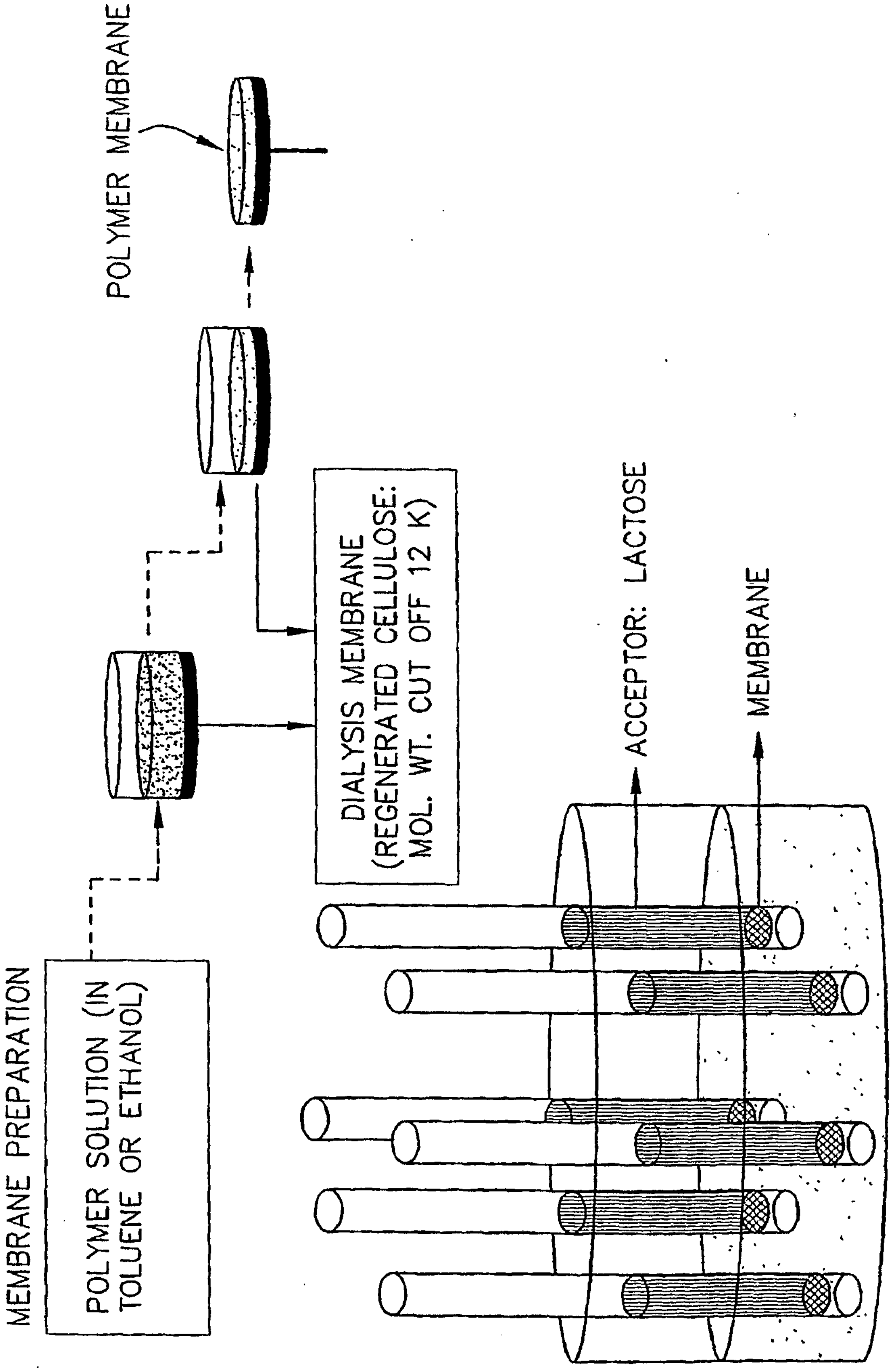
MEMBRANE PREPARATION
POLYMER SOLUTION (IN TOLUENE OR ETHANOL)
DIALYSIS MEMBRANE (REGENERATED CELLULOSE: MOL. WT. CUT OFF 12 K)
POLYMER MEMBRANE
ACCEPTOR: LACTOSE
MEMBRANE

FIG. 12

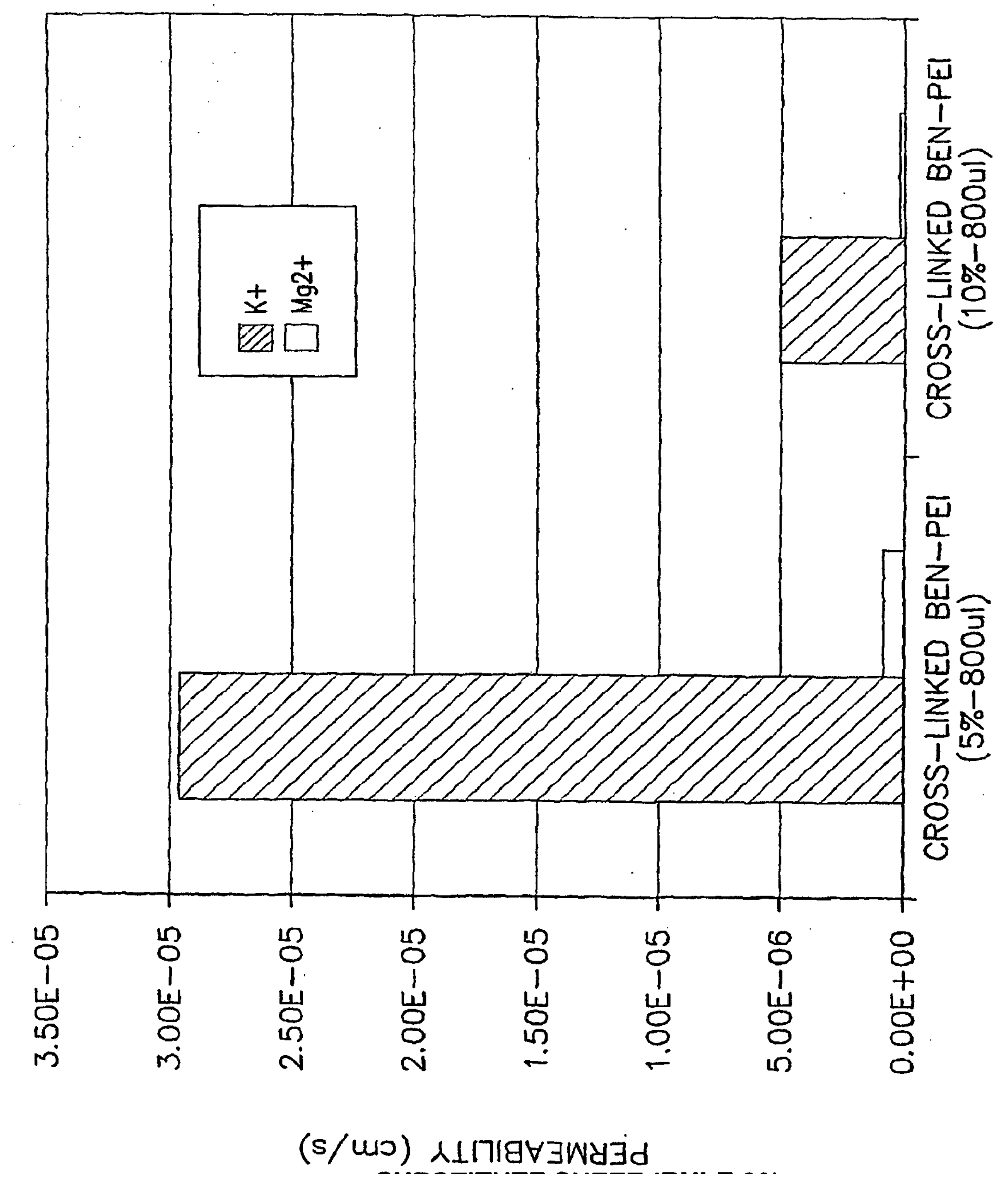
K+
Mg2+
3.50E−05
3.00E−05
2.50E−05
2.00E−05
1.50E−05
1.00E−05
5.00E−06
0.00E+00
PERMEABILITY (cm/s)
CROSS−LINKED BEN−PEI (5%−800ul)
CROSS−LINKED BEN−PEI (10%−800ul)
MEMBRANE

FIG. 13

FIG. 14A

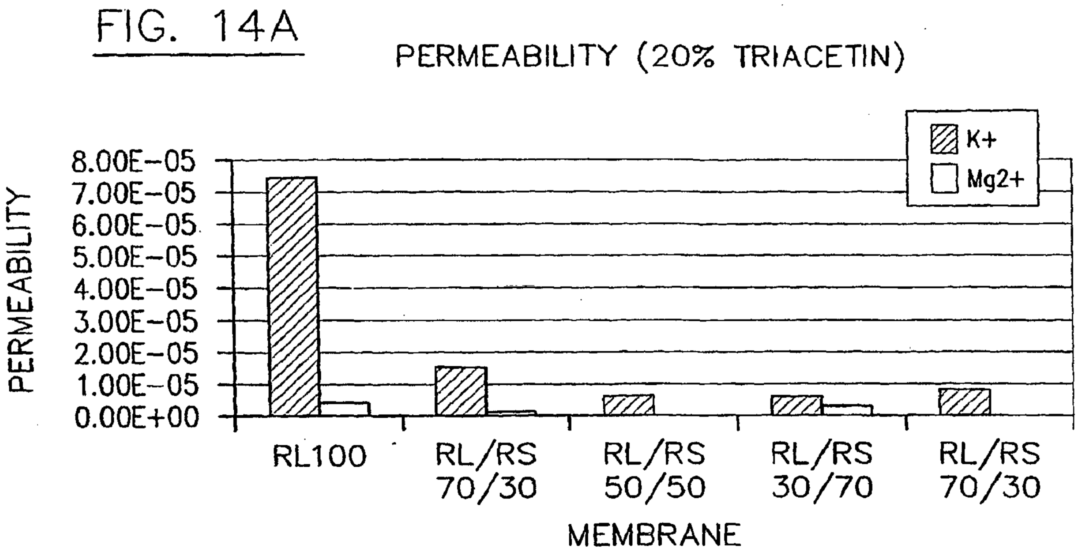


FIG. 14B

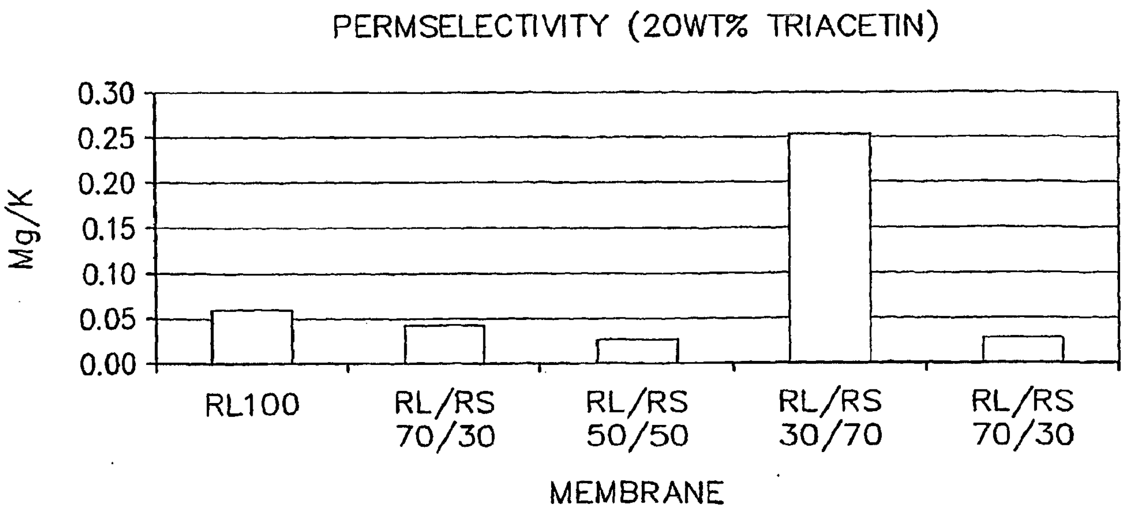

FIG. 15A

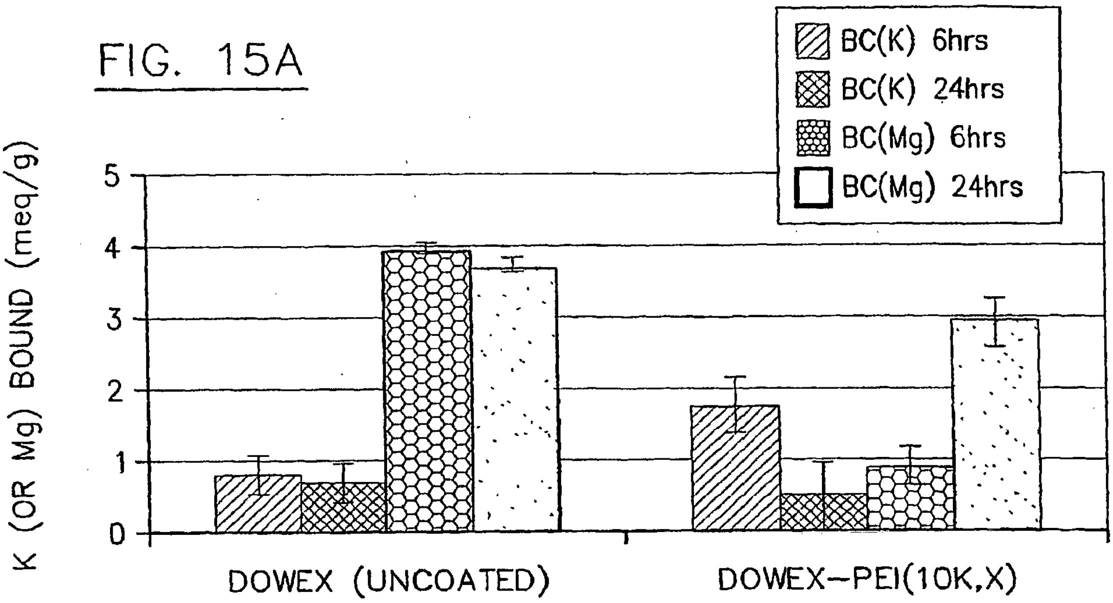


FIG. 15B

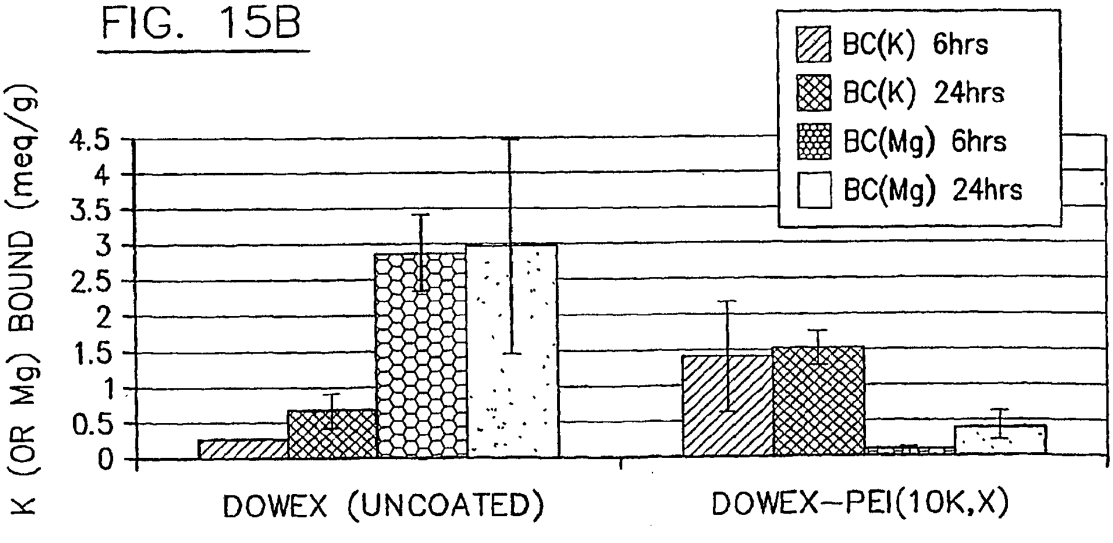

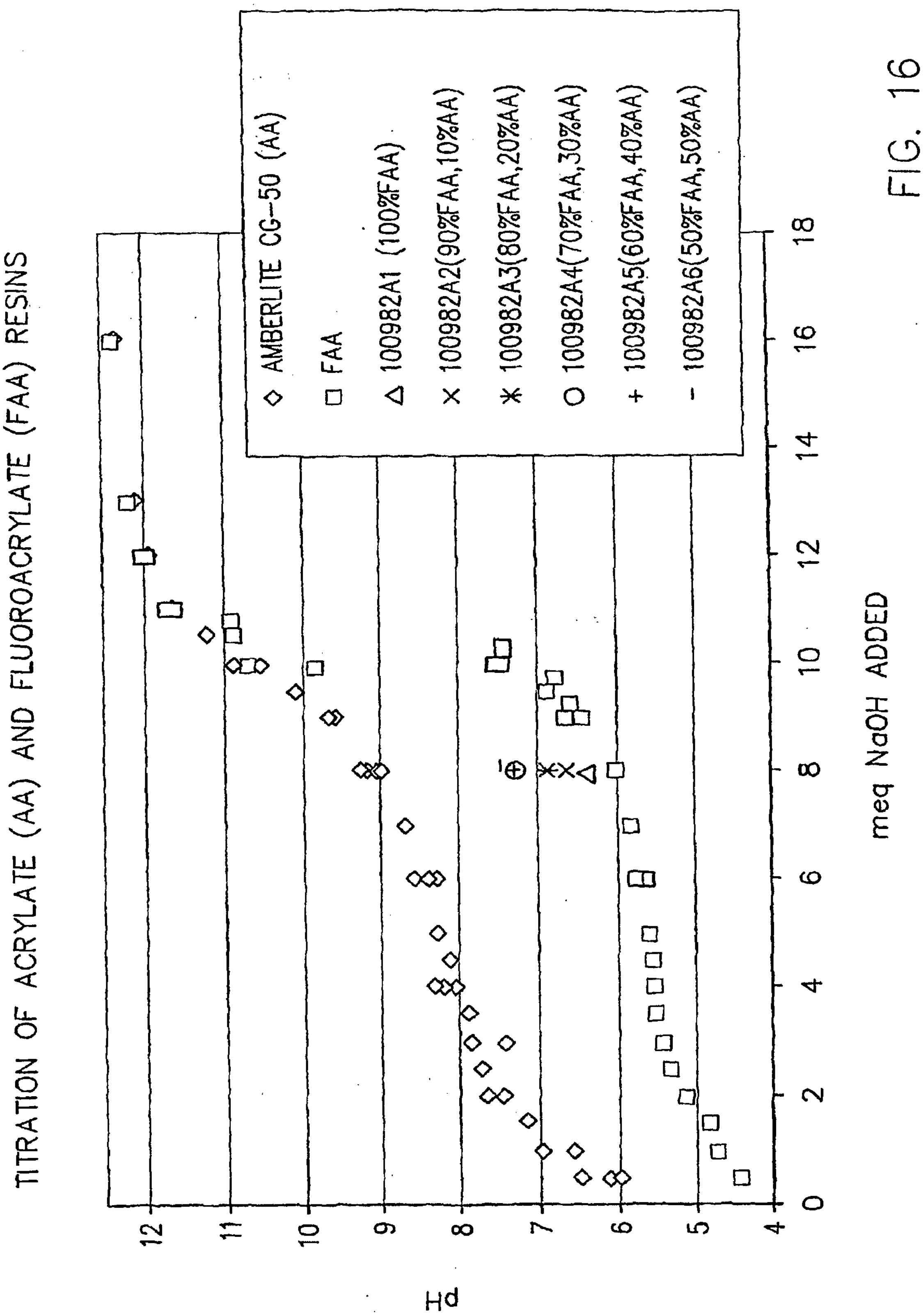
TITRATION OF ACRYLATE (AA) AND FLUOROACRYLATE (FAA) RESINS
◇ AMBERLITE CG–50 (AA)
□ FAA
△ 100982A1 (100%FAA)
× 100982A2(90%FAA,10%AA)
✳ 100982A3(80%FAA,20%AA)
○ 100982A4(70%FAA,30%AA)
+ 100982A5(60%FAA,40%AA)
− 100982A6(50%FAA,50%AA)
pH
12
11
10
9
8
7
6
5
4
0
2
4
6
8
10
12
14
16
18
meq NaOH ADDED

FIG. 16

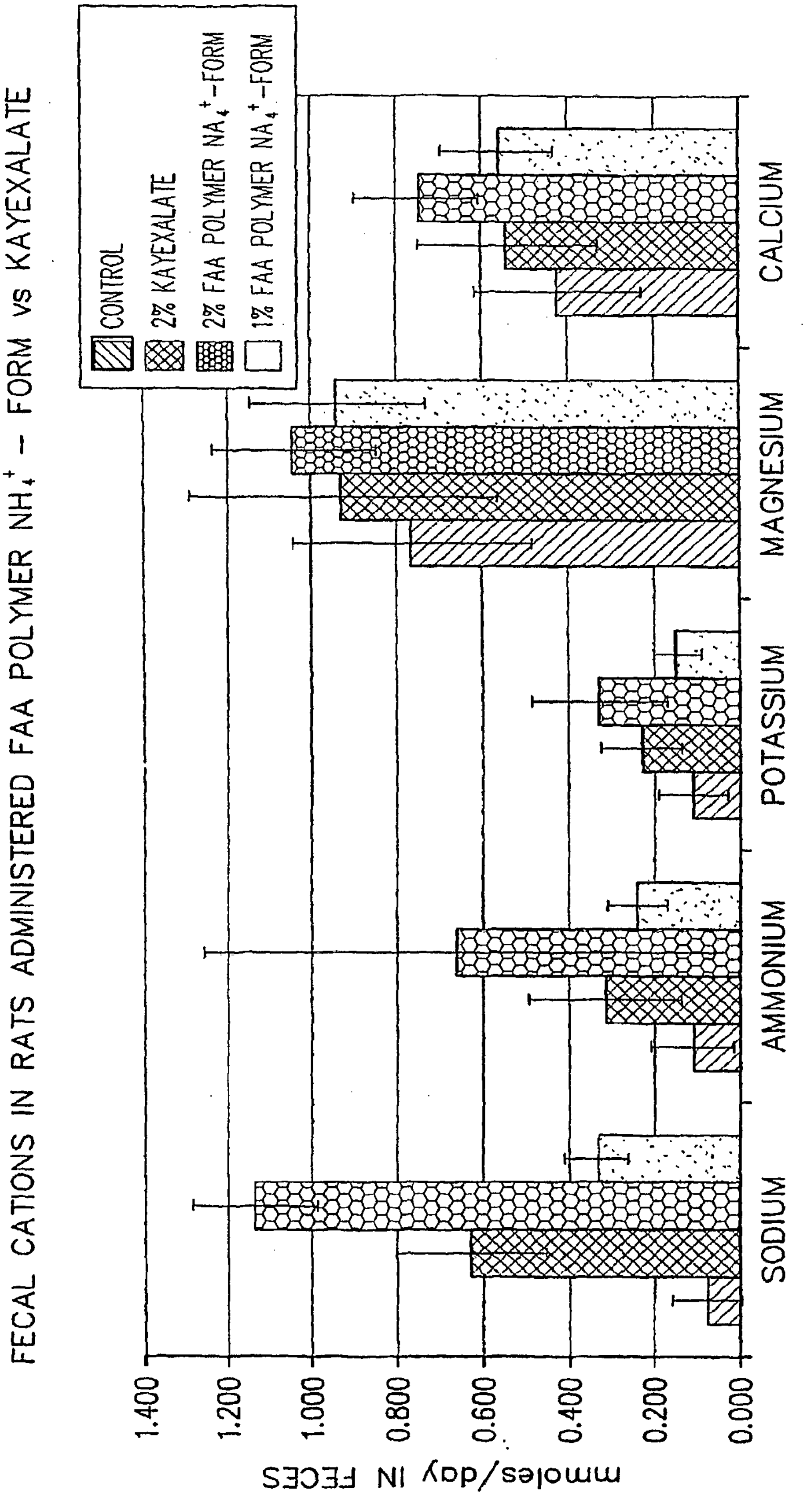
FECAL CATIONS IN RATS ADMINISTERED FAA POLYMER NH4+ – FORM vs KAYEXALATE
CONTROL
2% KAYEXALATE
2% FAA POLYMER NA4+-FORM
1% FAA POLYMER NA4+-FORM
mmoles/day IN FECES
1.400
1.200
1.000
0.800
0.600
0.400
0.200
0.000
SODIUM
AMMONIUM
POTASSIUM
MAGNESIUM
CALCIUM

FIG. 17

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5112993 A
- EP 415214 A
- US 4427794 A
- EP 373852 A2
- US 6475610 B


### Non-patent literature cited in the description


- **AGARWAL, R et al.** *Gastroenterology,* 1994, vol. 107, 548-571
- **MANDAL, AK.** *Med Clin North Am,* 1997, vol. 81, 611-639
- **HUNT, CD ; MEACHAM, SL.** *J Am Diet Assoc,* 2001, vol. 101, 1058-1060
- **HAZELL, T.** *World Rev Nutr Diet,* 1985, vol. 46, 1-123
- **SCHWARTZ, MW.** *Am J Nurs,* 1987, vol. 87, 1292-1299
- **KIM HO-JUNG et al.** Therapeutic Approach to Hyperkalemia. 01 October 2002, 33-40
- **GASSEN et al.** *J. Fluorine Chemistry,* 1991, vol. 55, 149-162
- **KF PITTMAN ; M. UEDA et al.** *Macromolecules,* 1980, vol. 13 (5), 1031-1036
- **BOGACHEV et al.** *Zhurnal Organisheskoi Khimii,* 1986, vol. 22 (12), 2578-83
- Nobel Lecture. The discovery of crown ethers. **CHARLES J. PEDERSON.** Nobel Lectures, Chemistry. World Scientific Publishing Co, 1981
- **MANDAL, A.K.** Hypokalemia and hyperkalemia. *Med Clin North Am.,* 1997, vol. 81, 611-39
- Remington's Pharmaceutical Sciences
- **BRESLOW et al.** *J. Am. Chem. Soc.,* 1954, vol. 76, 6399-6401
- **BADESSO et al.** *Hydrophilic Polymers: Performance with Environmental acceptance,* 489-504
- **G. VAN DEN MOOTER ; C. SAMYN ; R. KINGET.** *International Journal of Pharmaceutics,* 1994, vol. 111, 127-136